# EUROPEAN PATENT SPECIFICATION

(11) **EP 1 500 704 B1**
(45) Date of publication and mention of the grant of the patent: **25.03.2009**
(21) Application number: 03723103.2
(22) Date of filing: 11.04.2003
(51) Int. Cl.: C12N 15/70, C12N 15/53, C12N 15/12, C12P 7/62, C12N 1/21, C12R 1/04, C12R 1/38, C12R 1/465, C12R 1/19

(54) **EXPRESSION SYSTEM OF ACTINOMYCETE-ORIGIN CYTOCHROME P-450 IN ESCHERICHIA COLI**
EXPRESSIONSSYSTEM DES AUS ACTINOMYCETEN STAMMENDEN CYTOCHROM P-450 IN ESCHERICHIA COLI
SYSTEME D'EXPRESSION DE CYTOCHROME P-450 D'ORIGINE ACTINOMYCETE DANS DES ESCHERICHIA COLI

(30) Priority: 12.04.2002 JP 2002110311
(43) Date of publication of application: 26.01.2005
(73) Proprietor: MERCIAN CORPORATION, Chuo-ku, Tokyo 104-8305 (JP)
(72) Inventor: ARISAWA, Akira, Iwata-shi, Shizuoka 438-0078 (JP); KUMEDA, Ayako, Tokyo 140-0011 (JP)
(74) Representative: Hiebl, Inge Elisabeth
(86) International application number: PCT/JP2003/004609
(87) International publication number: WO 2003/087381

(56) References cited:
- EP-A- 1 270 722
- WO-A-93/12236
- WO-A-02/092801
- WO-A-03/057830
- WO-A1-93/12236
- WO-A1-97/19917
- JP-A- 7 067 666
- TAYLOR MARK ET AL: "Cytochrome P450105D1 (CYP105D1) from Streptomyces griseus: Heterologous expression, activity, and activation effects of multiple xenobiotics" BIOCHEMICAL AND BIOPHYSICAL RESEARCH COMMUNICATIONS, vol. 263, no. 3, 5 October 1999 (1999-10-05), pages 838-842, XP002366461 ISSN: 0006-291X
- PATENT ABSTRACTS OF JAPAN vol. 1995, no. 06, 31 July 1995 (1995-07-31) -& JP 07 067666 A (SUMITOMO CHEM CO LTD), 14 March 1995 (1995-03-14)
- PETERSON J A ET AL: "Putidaredoxin reductase and putidaradoxin. Cloning, sequence determination and heterologous expression of the proteins" JOURNAL OF BIOLOGICAL CHEMISTRY, AMERICAN SOCIETY OF BIOLOCHEMICAL BIOLOGISTS, BIRMINGHAM,, US, vol. 265, no. 11, 1990, pages 6066-6073, XP002968855 ISSN: 0021-9258
- NAGY I ET AL: "Degradation of the thiocarbamate herbicide EPTC (S-ethyl dipropylcarbamothioate) and biosafening by Rhodococcus sp. strain NI86/21 involve an inducible cytochrome P-450 system and aldehyde dehydrogenase" JOURNAL OF BACTERIOLOGY, WASHINGTON, DC, US, vol. 177, no. 3, February 1995 (1995-02), pages 676-687, XP002185660 ISSN: 0021-9193
- ZOTCHEV S B ET AL: "Cloning and heterologous expression of the genes encoding nonspecific electron transport components for a cytochrome P450 system of Saccharopolyspora erythraea involved in erythromycin production" GENE, ELSEVIER, AMSTERDAM, NL, vol. 156, no. 1, 14 April 1995 (1995-04-14), pages 101-106, XP004042396 ISSN: 0378-1119
- KOGA H ET AL: "CLONING AND NUCLEOTIDE SEQUENCES OF NADH PUTIDAREDOXIN REDUCTASE GENE CAM-A AND PUTIDAREDOXIN GENE CAM-B INVOLVED IN CYTOCHROME P-450-CAM HYDROXYLASE OF PSEUDOMONAS-PUTIDA" JOURNAL OF BIOCHEMISTRY (TOKYO), vol. 106, no. 5, 1989, pages 831-836, XP002366462 ISSN: 0021-924X
- DATABASE EMBL [Online] 28 July 1999 (1999-07-28), "Streptomyces coelicolor A3(2) dnaG gene and flanking genes" XP002366681 retrieved from EBI accession no. EM_PRO:SCO244019 Database accession no. SCO244019
- HUSSAIN HAITHAM A ET AL: "Enhanced heterologous expression of two Streptomyces griseolus cytochrome P450s and Streptomyces coelicolor ferredoxin reductase as potentially efficient hydroxylation catalysts." APPLIED AND ENVIRONMENTAL MICROBIOLOGY, vol. 69, no. 1, January 2003 (2003-01), pages 373-382, XP002366463 ISSN: 0099-2240
- PETERSON J.A. ET AL.: 'Putidaredoxin reductase and putidaradoxin. Cloning, sequence determination and heterologous expression of the proteins' J. BIOL. CHEM. vol. 265, no. 11, 1990, pages 6066 - 6073, XP002968855
- SIBBESEN O; ET AL: "Putidaredoxin reductase-putidaredoxin-cytochrome P450cam triple fusion protein" JOURNAL OF BIOLOGICAL CHEMISTRY, vol. 271, no. 37, - 13 September 1996 (1996-09-13) pages 22462-22469, XP002131693 BIRMINGHAM, US ISSN: 0021-9258

### SEQUENCE LISTING

<110> Mercian Corporation
<120> Expression system of Actinomycete-origin cytochrome
   P-450 in Escherichia coli
<130> K-43Mercian
<150> JP2002/110311
   <151> 2002-04-12
<160> 24
<210> 1
   <211> 2696
   <212> DNA
   <213> Microtetraspora recticatena IF014525
<220>
   <221> CDS
   <222> (313)..(1533)
<220>
   <221> CDS
   <222> (1547)..(1741)
<400>. 1
<210> 2
   <211> 1992
   <212> DNA
   <213> Streptomyces sp.TM-7
<220>
   <221> CDS
   <222> (544).. (1758)
<220>
   <221> CDS
   <222> (1782)..(1970)
<400> 2
   tcgccgggcc cggcggtgtg gaccgttcgc ggaccagccg ggcgaattcg gggtcgtgca 60
   tgacctcggt gagcaggccg cggagtatgt ccgccgtgcg aggcggccaa cctggcggag 120
   agtcgccgta gcgcggtgat gacatcggtg cgcagggcgc cggtgtcggg caggtcggcg 180
   tcggacagcc ggtgggccgc gcaggcgtcg acgacgagtt cggcacggcc gggccacctc 240
   cggtacaggg tggccttgcc ggtacgggcc cgtgcggcca cgcgctccat cgtcagtccc 300
   ggcgtagtcc gacctcggtc agtttcctcg agggtcgcgg ccaggatggc cctctccagt 360
   tcctctcctc ggccggcgag ggtttttcga tggtcgcggt cgtccggtcc ggcgcgtccc 420
   cgtgggttgg aggcatgact cccagccatt tgtcgagcac ccgttgtgag cgtcgggtgg 480
   gtaagcctag ccttccgtta gagaactgac cgttctttaa gcgtcgagtg catcgaggga 540
   ccg atg acc gag acc gtt acg acg ccc aca tca ggc gcc ccc gcc ttc 588
   Met Thr Glu Thr Val Thr Thr Pro Thr Ser Gly Ala Pro Ala Phe
   1 5 10 15
   ccc agt gac cgc acc tgc ccc tac cac ctc ccc gac cgg tac aac gac 636
   Pro Ser Asp Arg Thr Cys Pro Tyr His Leu Pro Asp Arg Tyr Asn Asp
   20 25 30
   ctc cgg gac cgg gag ggt tcg ctg cag cgg gtc acc ctc tac gac ggc 684
   Leu Arg Asp Arg Glu Gly Ser Leu Gln Arg Val Thr Leu Tyr Asp Gly
   35 40 45
   cgg cag gca tgg ctg gtg acc ggg tac gac acg gca cgc aag ctg ctg 732
   Arg Gln Ala Trp Leu Val Thr Gly Tyr Asp Thr Ala Arg Lys Leu Leu
   50 55 60
   gcc gac ccc cgg ctc tcg tcc gac cgg aca cac gcc gac ttc ccc gcc 780
   Ala Asp Pro Arg Leu Ser Ser Asp Arg Thr His Ala Asp Phe Pro Ala
   65 70 75
   acc tcc ggg cgg gtg gag agc ttc cgg gac cgc cgg ccg gcg ttc atc 828
   Thr Ser Gly Arg Val Glu Ser Phe Arg Asp Arg Arg Pro Ala Phe Ile
   80 85 90 95
   agc ctg gac ccg ccc gag cac ggg ccg aaa cgg cgc cat gac cat cag 876
   Ser Leu Asp Pro Pro Glu His Gly Pro Lys Arg Arg His Asp His Gln
   100 105 110
   gag ttc acc gtc cgg cgc atc aag ggc atg cgg gcc gac gtc gag cag 924
   Glu Phe Thr Val Arg Arg Ile Lys Gly Met Arg Ala Asp Val Glu Gln
   115 120 125
   atc gtg cac ggc ttc ctg gac gag atg atc gca ggc ggc ccg ccc gcc 972
   Ile Val His Gly Phe Leu Asp Glu Met Ile Ala Gly Gly Pro Pro Ala
   130 135 140
   gac ctg gtc agc cag ttc gcg ctg ccc gtc ccg tcc ctg gtg atc tgc 1020
   Asp Leu Val Ser Gln Phe Ala Leu Pro Val Pro Ser Leu Val Ile Cys
   145 150 155
   cgt ctg ctc ggt gtg ccc tac gcg gac cac gac ttc ttc cag gac gcc 1068
   Arg Leu Leu Gly Val Pro Tyr Ala Asp His Asp Phe Phe Gln Asp Ala
   160 165 170 175
   agc gca cgg ctg atc cag tcc ccg gac gcg gcg ggt gcg cgt gcc gcc 1116
   Ser Ala Arg Leu Ile Gln Ser Pro Asp Ala Ala Gly Ala Arg Ala Ala
   180 185 190
   cgg gac gac ctg gag agc tat ctg ggc gct ctg gtg gac agc ctg cga 1164
   Arg Asp Asp Leu Glu Ser Tyr Leu Gly Ala Leu Val Asp Ser Leu Arg
   195 200 205
   ggc gag tcc cgg ccg ggc ctg ctg agc acg ctc gtc agg gag cag ctg 1212
   Gly Glu Ser Arg Pro Gly Leu Leu Ser Thr Leu Val Arg Glu Gln Leu
   210 215 220
   gag aag ggc gcg atc gac cgg gag gag ctg gtg tcg acg gcg atc ctg 1260
   Glu Lys Gly Ala Ile Asp Arg Glu Glu Leu Val Ser Thr Ala Ile Leu
   225 230 235
   ctg ctg gtc gcc gga cac gag gcg acg gcg tcg atg acg tcg ctc agc 1308
   Leu Leu Val Ala Gly His Glu Ala Thr Ala Ser Met Thr Ser Leu Ser
   240 245 250 255
   gtc atc acc ctc ctc gaa cat ccc gac cag cac gcc gcg ttg cgc gcc 1356
   Val Ile Thr Leu Leu Glu His Pro Asp Gln His Ala Ala Leu Arg Ala
   260 265 270
   gat ccg tcg ctg gtg ccc ggc gcg gtg gag gag ctg ctg cgc tat ctg 1404
   Asp Pro Ser Leu Val Pro Gly Ala Val Glu Glu Leu Leu Arg Tyr Leu
   275 280 285
   gcc atc gcc gac atc gcc ggc ggg cgg atc gcg acg gcg gac atc gag 1452
   Ala Ile Ala Asp Ile Ala Gly Gly Arg Ile Ala Thr Ala Asp Ile Glu
   290 295 300
   atc gac ggg cag cgc atc cgg gcg ggg gag ggg gtc atc gtc acc aac 1500
   Ile Asp Gly Gln Arg Ile Arg Ala Gly Glu Gly Val Ile Val Thr Asn
   305 310 315
   tcg atc gcc aac cgc gac ggc tcc gtc ttc gcc gac ccg gac gcc ttc 1548
   Ser Ile Ala Asn Arg Asp Gly Ser Val Phe Ala Asp Pro Asp Ala Phe
   320 325 330 335
   gac gtg cgg cgc gag gcc cgc cac cac ctg gcg ttc ggc tac ggg gtg 1596
   Asp Val Arg Arg Glu Ala Arg His His Leu Ala Phe Gly Tyr Gly Val
   340 345 350
   cat cag tgc ctc ggc cag aac ctg gcc cgc ctc gaa ctg gag gtc atc 1644
   His Gln Cys Leu Gly Gln Asn Leu Ala Arg Leu Glu Leu Glu Val Ile
   355 360 365
   ctc acg gcg ctg ttc gag cgg ctg ccc ggt ctg cgg ctg gcg gtg ccg 1692
   Leu Thr Ala Leu Phe Glu Arg Leu Pro Gly Leu Arg Leu Ala Val Pro
   370 375 380
   gtg gac cgg ctg acc ctg cgc ccg ggc acg acg atc cag ggc gtg aac 1740
   Val Asp Arg Leu Thr Leu Arg Pro Gly Thr Thr Ile Gln Gly Val Asn
   385 390 395
   gaa ctc ccg gtc acc tgg tgaccgcggc gaaaggagca gcc atg cgt gtg acg 1793
   Glu Leu Pro Val Thr Trp Met Arg Val Thr
   400 405
   gcc gac cgg gag gtc tgc gtg gga gcg ggc ctg tgc gcc ttg acg gcg 1841
   Ala Asp Arg Glu Val Cys Val Gly Ala Gly Leu Cys Ala Leu Thr Ala
   410 415 420 425
   ccg gag gtc ttc gac cag gac gac gac ggt gtg gtg acg gtg ctg gcc 1889
   Pro Glu Val Phe Asp Gln Asp Asp Asp Gly Val Val Thr Val Leu Ala
   430 435 440
   gcg gaa ccc ggc gag gcc ggc cgt gcg gcg gca ctc gaa gcc ggc gtg 1937
   Ala Glu Pro Gly Glu Ala Gly Arg Ala Ala Ala Leu Glu Ala Gly Val
   445 450 455
   ctg tgc ccg tcc ggc gcg gta cgc gtc gtc gag taggggccgt gcggggccgt 1990
   Leu Cys Pro Ser Gly Ala Val Arg Val Val Glu
   460 465
   ga 1992
<210> 3
   <211> 45
   <212> DNA
   <213> Artificial Sequence
<220>
   <223> STRANDNESS:single
<220>
   <223> TOPOLOGY:linear
<220>
   <223> Description of Artificial Sequence:FDR1-1F Primer
<400> 3
   gccatatgac tagtgcgcct cacagactgg aacgggaatc tcatg 45
<210> 4
   <211> 38
   <212> DNA
   <213> Artificial Sequence
<220>
   <223> STRANDNESS:single
<220>
   <223> TOPOLOGY:linear
   <223> Description of Artificial Sequence:FDR1-2R Primer
<400> 4
   gcgaattctg tcggtcaggc ctggtctccc gtcggccg 38
<210> 5
   <211> 1439
   <212> DNA
   <213> Streptomyces coelicolor A3(2)
<220>
   <221> CDS
   <222> (118)..(1377)
<400> 5
   gatcacgggg gccgggtagg cccgtgccac ggtgtcacaa cgcgtgtcgt cgcccggtct 60
   gaaggatgac cggaccactc ggtccgtgtg cgcctcacag actggaacgg gaatctc atg 120
   Met
   1
   ccg cgt gcg aag acg ttc gtg atc gtc ggg ggc ggc ctg gcc gcc ggc 168
   Pro Arg Ala Lys Thr Phe Val Ile Val Gly Gly Gly Leu Ala Ala Gly
   5 10 15
   aag gcc gcg gag gaa ctg cgc gag cac ggc cac gac ggg ccg ctt ctc 216
   Lys Ala Ala Glu Glu Leu Arg Glu His Gly His Asp Gly Pro Leu Leu
   20 25 30
   gtg atc ggg gac gag cgg gaa cga ccg tac atc cgg ccg ccg ctg tcc 264
   Val Ile Gly Asp Glu Arg Glu Arg Pro Tyr Ile Arg Pro Pro Leu Ser
   35 40 45
   aag ggg tac ctg ctg ggc aag gag gac cgc gag tcc atc cac gtg cac 312
   Lys Gly Tyr Leu Leu Gly Lys Glu Asp Arg Glu Ser Ile His Val His
   50 55 60 65
   ccc gag agc tgg tac cgg gag cac gac gtc gat ctg ctc ctc ggc acg 360
   Pro Glu Ser Trp Tyr Arg Glu His Asp Val Asp Leu Leu Leu Gly Thr
   70 75 80
   agc gtg acg tcc gtc gac gcg cgt ggc cgg gcg gtg acg ctg gac gac 408
   Ser Val Thr Ser Val Asp Ala Arg Gly Arg Ala Val Thr Leu Asp Asp
   85 90 95
   ggc cgt cgc gtg ccc tac gcc ggt ctg ctg ctg gcc acc ggt tcc tcg 456
   Gly Arg Arg Val Pro Tyr Ala Gly Leu Leu Leu Ala Thr Gly Ser Ser
   100 105 110
   ccg cgc cgc ctg tcg gtg ccg ggc gcg gac ctg gag ggc gtg ctg tac 504
   Pro Arg Arg Leu Ser Val Pro Gly Ala Asp Leu Glu Gly Val Leu Tyr
   115 120 125
   ctg cgg cgc gtg ggc gac agc gag cgc ctc aag gag gcg ttc acc gaa 552
   Leu Arg Arg Val Gly Asp Ser Glu Arg Leu Lys Glu Ala Phe Thr Glu
   130 135 140 145
   gga gcc cgg atc gtg gtg gtc ggc ggc ggc tgg atc ggg ctg gag acg 600
   Gly Ala Arg Ile Val Val Val Gly Gly Gly Trp Ile Gly Leu Glu Thr
   150 155 160
   gcg gcg gcg gcc cgg gcg gcc ggc gcg gag gtg acc gtg ctg gag cgc 648
   Ala Ala Ala Ala Arg Ala Ala Gly Ala Glu Val Thr Val Leu Glu Arg
   165 170 175
   ggt gag ctg ccc ctg ctg aag gtc ctg ggc cgc gag gcg gcc gag gtc 696
   Gly Glu Leu Pro Leu Leu Lys Val Leu Gly Arg Glu Ala Ala Glu Val
   180 185 190
   ttc gcc ggt ctg cac cgg gac cac ggt gtg gac ctg cgt ccc cat gcc 744
   Phe Ala Gly Leu His Arg Asp His Gly Val Asp Leu Arg Pro His Ala
   195 200 205
   cgg atc gag gcc gtc acc ggc acc ggg ggc cgc gtc gac ggg gtc cgg 792
   Arg Ile Glu Ala Val Thr Gly Thr Gly Gly Arg Val Asp Gly Val Arg
   210 215 220 225
   ctc gcc gac ggc acc cac ctg ccc gcg gac gcc gtg gtc gtg ggg gtg 840
   Leu Ala Asp Gly Thr His Leu Pro Ala Asp Ala Val Val Val Gly Val
   230 235 240
   ggc atc acg ccc aac gtc cgc ctg gcc gag gag gcg ggc ctc gac gtg 888
   Gly Ile Thr Pro Asn Val Arg Leu Ala Glu Glu Ala Gly Leu Asp Val
   245 250 255
   cgc aac ggc atc gtg acg gac gcc cgt ctg cgg acc tcc gcc gcc ggg 936
   Arg Asn Gly Ile Val Thr Asp Ala Arg Leu Arg Thr Ser Ala Ala Gly
   260 265 270
   gtc cac gcc gcc ggt gac gtc gcc aac gcc tac cac ccc cgg ctc ggc 984
   Val His Ala Ala Gly Asp Val Ala Asn Ala Tyr His Pro Arg Leu Gly
   275 280 285
   cgg cac ctg cgc gtg gag cac tgg gcc aac gcg ctg cac cag ccc cgt 1032
   Arg His Leu Arg Val Glu His Trp Ala Asn Ala Leu His Gln Pro Arg
   290 295 300 305
   acc gcc gcg ctg agc atg ctc ggc cag gac gcg gtg tac gac cgg ctg 1080
   Thr Ala Ala Leu Ser Met Leu Gly Gln Asp Ala Val Tyr Asp Arg Leu
   310 315 320
   ccg tac ttc tac acc gac cag tac gac ctc ggc atg gag tac acc ggg 1128
   Pro Tyr Phe Tyr Thr Asp Gln Tyr Asp Leu Gly Met Glu Tyr Thr Gly
   325 330 335
   tac gcc gaa ccg ggc ggc tac gac cgc gtc gtc ttc cgc ggg tcg cgc 1176
   Tyr Ala Glu Pro Gly Gly Tyr Asp Arg Val Val Phe Arg Gly Ser Arg
   340 345 350
   gag gag cgg cgg ttc ctg gcg ttc tgg atg tcc ggc gac cgg gtg ctg 1224
   Glu Glu Arg Arg Phe Leu Ala Phe Trp Met Ser Gly Asp Arg Val Leu
   355 360 365
   gcg ggg atg agc gtc aac ctg tgg gac gtg atc ggg acg atc cgc gcc 1272
   Ala Gly Met Ser Val Asn Leu Trp Asp Val Ile Gly Thr Ile Arg Ala
   370 375 380 385
   ctg atc gag tcg ggc gcg gag acg gac gac gcc gcc ctg gcc gac ccc 1320
   Leu Ile Glu Ser Gly Ala Glu Thr Asp Asp Ala Ala Leu Ala Asp Pro
   390 395 400
   tcg gtc ccg ctg gag agc ctg ctt ccc ccg cac gcg cgg ccg acg gga 1368
   Ser Val Pro Leu Glu Ser Leu Leu Pro Pro His Ala Arg Pro Thr Gly
   405 410 415
   gac cag gcg tga ccgacagcgc tcccgcgccg ctgcgctcga tgccggacga 1420
   Asp Gln Ala
   420
   ctggcggcgg gccctggcc 1439
<210> 6
   <211> 43
   <212> DNA
   <213> Artificial Sequence
<220>
   <223> STRANDNESS:single
<220>
   <223> TOPOLOGY:linear
<220>
   <223> Description of Artificial Sequence:FDR2-3F Primer
<400> 6
   cgactagtga cgaggaggca gacaaatggt cgacgcggat cag 43
<210> 7
   <211> 36
   <212> DNA
   <213> Artificial Sequence
<220>
   <223> STRANDNESS:single
<220>
   <223> TOPOLOGY:linear
<220>
   <223> Description of Artificial Sequence:FDR2-4R Primer
<400> 7
   cgggatccga caactatgcg acgaggcttt cgaggg 36
<210> 8
   <211> 1319
   <212> DNA
   <213> Streptomyces coelicolor A3(2)
<220>
   <221> CDS
   <222> (34)..(1296)
<400> 8
   cacgtggcgg caccctgacg aggaggcaga caa gtg gtc gac gcg gat          48
                                        Met Val Asp Ala Asp
                                        1               5
   cag aca ttc gtc atc gtc gga ggc ggc ctg gcg ggc gcg aaa gcg gcc   96
   Gln Thr Phe Val Ile Val Gly Gly Gly Leu Ala Gly Ala Lys Ala Ala
   10 15 20
   gag acg ctc cgc acg gag ggc ttc acc ggc cgg gtg atc ctc gtc tgc 144
   Glu Thr Leu Arg Thr Glu Gly Phe Thr Gly Arg Val Ile Leu Val Cys
   25 30 35
   gac gaa cgc gac cac ccc tac gag cgc ccg ccg ctg tcc aag ggc tac 192
   Asp Glu Arg Asp His Pro Tyr Glu Arg Pro Pro Leu Ser Lys Gly Tyr
   40 45 50
   ctc ctg ggc aag gag gag cgc gac agc gtc ttc gtg cac gag ccc gcc 240
   Leu Leu Gly Lys Glu Glu Arg Asp Ser Val Phe Val His Glu Pro Ala
   55 60 65
   tgg tac gcc cgg cac gac atc gag ctg cac ctc ggc cag acc gtc gtc 288
   Trp Tyr Ala Arg His Asp Ile Glu Leu His Leu Gly Gln Thr Val Val
   70 75 80 85
   gcg atc gac cgc gcc gcc aag acc gtc cac tac ggc gac gac ggc acc 336
   Ala Ile Asp Arg Ala Ala Lys Thr Val His Tyr Gly Asp Asp Gly Thr
   90 95 100
   cac gtc agc tac gac aag ctg ctc atc gcg acc ggc gcc gag ccc cgc 384
   His Val Ser Tyr Asp Lys Leu Leu Ile Ala Thr Gly Ala Glu Pro Arg
   105 110 105
   cgc ctg gac gtc ccc ggc acc ggc ctc gcg ggc gtc cac cac ctg cgc 432
   Arg Leu Asp Val Pro Gly Thr Gly Leu Ala Gly Val His His Leu Arg
   110 115 120
   cgc ctg gcg cac gcc gag cgc ctc aag ggc gtc ctc gcc acc ctc ggc 480
   Arg Leu Ala His Ala Glu Arg Leu Lys Gly Val Leu Ala Thr Leu Gly
   125 130 135
   cgg gac aac gga cac ctg gtg atc gcc ggc gcg ggc tgg atc ggc ctg 528
   Arg Asp Asn Gly His Leu Val Ile Ala Gly Ala Gly Trp Ile Gly Leu
   140 145 150 155
   gag gtc gcg gcc gcg gcc cgc gag tac ggt gcg gag gtc acc gtc atc 576
   Glu Val Ala Ala Ala Ala Arg Glu Tyr Gly Ala Glu Val Thr Val Ile
   160 165 170
   gag ccc gcc ccg acc ccg ctg cac ggc gtc ctc ggt ccc gag ctg ggc 624
   Glu Pro Ala Pro Thr Pro Leu His Gly Val Leu Gly Pro Glu Leu Gly
   175 180 185
   gcc gtc ttc gcc gag ctg cac gag tcg cgc ggc gtc cgc ttc cgc ttc 672
   Ala Val Phe Ala Glu Leu His Glu Ser Arg Gly Val Arg Phe Arg Phe
   190 195 200
   ggc gtg aag ctg acc gag atc gtc ggc cag gac ggt gtg gtg ctg gcc 720
   Gly Val Lys Leu Thr Glu Ile Val Gly Gln Asp Gly Val Val Leu Ala
   205 210 205
   gcc cgc acc gac gac ggc gag gag cac ccc gcg cac gac gtg ctc gcc 768
   Ala Arg Thr Asp Asp Gly Glu Glu His Pro Ala His Asp Val Leu Ala
   210 215 220 225
   gcg atc ggc gcc gcc ccg cgc acc gcg ctc gcc cag gcg gcc ggg ttg 816
   Ala Ile Gly Ala Ala Pro Arg Thr Ala Leu Ala Gln Ala Ala Gly Leu
   330 335 340
   gag atc gcc gac cgc gcg cac ggc ggc ggc atc gtc gtc gac gac cac 864
   Glu Ile Ala Asp Arg Ala His Gly Gly Gly Ile Val Val Asp Asp His
   345 350 355
   ctg cgc acc tcc gac ccc gac atc ttc gcg gcc ggc gac gtg gcc tcc 912
   Leu Arg Thr Ser Asp Pro Asp Ile Phe Ala Ala Gly Asp Val Ala Ser
   360 365 370
   ttc cac cac gcc ctc ttc gac acc agc ctg cgc gtg gag cac tgg gcc 960
   Phe His His Ala Leu Phe Asp Thr Ser Leu Arg Val Glu His Trp Ala
   375 380 385
   aac gcc ctg aac ggc ggt ccg gcc gcc gcc cgc gcg atg ctc ggc agg 1008
   Asn Ala Leu Asn Gly Gly Pro Ala Ala Ala Arg Ala MET Leu Gly Arg
   390 395 400 405
   ggc ctc gcc cac gac cgc gtg ccc tac ttc ttc acc gac cag tac gac 1056
   Gly Leu Ala His Asp Arg Val Pro Tyr Phe Phe Thr Asp Gln Tyr Asp
   410 415 420
   ctg ggc atg gag tac tcc ggc tgg gcg ccg gcc ggc tcg tac gac cag 1104
   Leu Gly MET Glu Tyr Ser Gly Trp Ala Pro Ala Gly Ser Tyr Asp Gln
   425 430 435
   gtg gtg atc cgc ggg gac gcg gcg aag cgc gag ttc atc gcc ttc tgg 1152
   Val Val Ile Arg Gly Asp Ala Ala Lys Arg Glu Phe Ile Ala Phe Trp
   440 445 450
   gtg aag gag ggc cgg gtg ctg gcc ggg atg aac gtc aac gtg tgg gac 1200
   Val Lys Glu Gly Arg Val Leu Ala Gly MET Asn Val Asn Val Trp Asp
   455 460 465
   gtc acg gag ccg atc cag cag ctg atc cgc tcg aag acc cgg gtg gac 1248
   Val Thr Glu Pro Ile Gln Gln Leu Ile Arg Ser Lys Thr Arg Val Asp
   470 475 480 485
   acg gag gac ctg gcg aac ccg cac gta tcc ctc gaa agc ctc gtc gca 1296
   Thr Glu Asp Leu Ala Asn Pro His Val Ser Leu Glu Ser Leu Val Ala
   490 505 510
   tagttgtcgg tccgcccccg tag 1319
<210> 9
   <211> 36
   <212> DNA
   <213> Artificial Sequence
<220>
   <223> STRANDNESS:single
<220>
   <223> TOPOLOGY:linear
<220>
   <223> Description of Artificial Sequence:PRR-1F Primer
<400> 9
   gccccccata tgaacgcaaa cgacaacgtg gtcatc 36
<210> 10
   <211> 35
   <212> DNA
   <213> Artificial Sequence
<220>
   <223> STRANDNESS:single
<220>
   <223> TOPOLOGY:linear
<220>
   <223> Description of Artificial Sequence:PRR-2R Primer
<400> 10
   gcggatcctc aggcactact cagttcaget ttggc 35
<210> 11
   <211> 36
   <212> DNA
   <213> Artificial Sequence
   <220>
   <223> STRANDNESS:single
<220>
   <223> TOPOLOGY:linear
<220>
   <223> Description of Artificial Sequence:Mox-1F Primer
<400> 11
   gcccccccata tgacgaagaa cgtcgccgac gaactg 36
<210> 12
   <211> 35
   <212> DNA
   <213> Artificial Sequence
<220>
   <223> STRANDNESS:single
<220>
   <223> TOPOLOGY:linear
<220>
   <223> Description of Artificial Sequence:Mox-12R Primer
<400> 12
   gcagatctag tggcttcagg cgtcccgcag gatgg 35
<210> 13
   <211> 32
   <212> DNA
   <213> Artificial Sequence
<220>
   <223> STRANDNESS:single
<220>
   <223> TOPOLOGY:linear
<220>
   <223> Description of Artificial Sequence: Mox-2R Primer
<400> 13
   cgactagtgg cttcaggcgt cccgcaggat gg 32
<210> 14
   <211> 24
   <212> DNA
   <213> Artificial Sequence
<220>
   <223> STRANDNESS:single
<220>
   <223> TOPOLOGY:linear
   <220>
   <223> Description of Artificial Sequence:Mox-3F Primer
<400> 14
   ggagatatac atatgacgaa gaac 24
<210> 15
   <211> 41
   <212> DNA
   <213> Artificial Sequence
<220>
   <223> STRANDNESS:single
<220>
   <223> TOPOLOGY:linear
<220>
   <223> Description of Artificial Sequence:Mox-5R Primer
<400> 15
   gccccccata tgacgcactc ctagtggctt caggcgtccc g 41
<210> 16
   <211> 1950
   <212> DNA
   <213> Pseudomonas putida ATCC17453
<220>
   <221> CDS
   <222> (115)..(1380)
<220>
   <221> CDS
   <222> (1439).. (1759)
<400> 16
   ccgggtgccc agattcagca caagagcggc atcgtcagcg gcgtgcaggc actccctctg 60
   gtctgggatc cggcgactac caaagcggta taaacacatg ggagtgcgtg ctaa gtg aac 120
   Met Asn
   1
   gca aac gac aac gtg gtc atc gtc ggt acc gga ctg gct ggc gtt gag 168
   Ala Asn Asp Asn Val Val Ile Val Gly Thr Gly Leu Ala Gly Val Glu
   5 10 15
   gtc gcc ttc ggc ctg cgc gcc agc ggc tgg gaa ggc aat atc cgg ttg 216
   Val Ala Phe Gly Leu Arg Ala Ser Gly Trp Glu Gly Asn Ile Arg Leu
   20 25 30
   gtg ggg gat gcg acg gta att ccc cat cac cta cca ccg cta tcc aaa 264
   Val Gly Asp Ala Thr Val Ile Pro His His Leu Pro Pro Leu Ser Lys
   35 40 45 50
   gct tac ttg gcc ggc aaa gcc aca gcg gaa agc ctg tac ctg aga acc 312
   Ala Tyr Leu Ala Gly Lys Ala Thr Ala Glu Ser Leu Tyr Leu Arg Thr
   55 60 65
   cca gat gcc tat gca gcg cag aac atc caa cta ctc gga ggc aca cag 360
   Pro Asp Ala Tyr Ala Ala Gln Asn Ile Gln Leu Leu Gly Gly Thr Gln
   70 75 80
   gta acg gct atc aac cgc gac cga cag caa gta atc cta tcg gat ggc 408
   Val Thr Ala Ile Asn Arg Asp Arg Gln Gln Val Ile Leu Ser Asp Gly
   85 90 95
   cgg gca ctg gat tac gac cgg ctg gta ttg gct acc gga ggg cgt cca 456
   Arg Ala Leu Asp Tyr Asp Arg Leu Val Leu Ala Thr Gly Gly Arg Pro
   100 105 110
   aga ccc cta ccg gtg gcc agt ggc gca gtt gga aag gcg aac aac ttt 504
   Arg Pro Leu Pro Val Ala Ser Gly Ala Val Gly Lys Ala Asn Asn Phe
   115 120 125 130
   cga tac ctg cgc aca ctc gag gac gcc gag tgc att cgc cgg cag ctg 552
   Arg Tyr Leu Arg Thr Leu Glu Asp Ala Glu Cys Ile Arg Arg Gln Leu
   135 140 145
   att gcg gat aac cgt ctg gtg gtg att ggt ggc ggc tac att ggc ctt 600
   Ile Ala Asp Asn Arg Leu Val Val Ile Gly Gly Gly Tyr Ile Gly Leu
   150 155 160
   gaa gtg gct gcc acc gcc atc aag gcg aac atg cac gtc acc ctg ctt 648
   Glu Val Ala Ala Thr Ala Ile Lys Ala Asn Met His Val Thr Leu Leu
   165 170 175
   gat acg gca gcc cgg gtt ctg gag cgg gtt acc gcc ccg ccg gta tcg 696
   Asp Thr Ala Ala Arg Val Leu Glu Arg Val Thr Ala Pro Pro Val Ser
   180 185 190
   gcc ttt tac gag cac cta cac cgc gaa gcc ggc gtt gac ata cga acc 744
   Ala Phe Tyr Glu His Leu His Arg Glu Ala Gly Val Asp Ile Arg Thr
   195 200 205 210
   ggc acg cag gtg tgc ggg ttc gag atg tcg acc gac caa cag aag gtt 792
   Gly Thr Gln Val Cys Gly Phe Glu Met Ser Thr Asp Gln Gln Lys Val
   215 220 225
   acc gcc gtc ctc tgc gag gac ggc aca agg ctg cca gcg gat ctg gta 840
   Thr Ala Val Leu Cys Glu Asp Gly Thr Arg Leu Pro Ala Asp Leu Val
   230 235 240
   atc gcc ggg att ggc ctg ata cca aac tgc gag ttg gcc agt gcg gcc 888
   Ile Ala Gly Ile Gly Leu Ile Pro Asn Cys Glu Leu Ala Ser Ala Ala
   245 250 255
   ggc ctg cag gtt gat aac ggc atc gtg atc aac gaa cac atg cag acc 936
   Gly Leu Gln Val Asp Asn Gly Ile Val Ile Asn Glu His Met Gln Thr
   260 265 270
   tct gat ccc ttg atc atg gcc gtc ggc gac tgt gcc cga ttt cac agt 984
   Ser Asp Pro Leu Ile Met Ala Val Gly Asp Cys Ala Arg Phe His Ser
   275 280 285 290
   cag ctc tat gac cgc tgg gtg cgt atc gaa tcg gtg ccc aat gcc ttg 1032
   Gln Leu Tyr Asp Arg Trp Val Arg Ile Glu Ser Val Pro Asn Ala Leu
   295 300 305
   gag cag gca cga aag atc gcc gcc atc ctc tgt ggc aag gtg cca cgc 1080
   Glu Gln Ala Arg Lys Ile Ala Ala Ile Leu Cys Gly Lys Val Pro Arg
   310 315 320
   gat gag gcg gcg ccc tgg ttc tgg tcc gat cag tat gag atc gga ttg 1128
   Asp Glu Ala Ala Pro Trp Phe Trp Ser Asp Gln Tyr Glu Ile Gly Leu
   325 330 335
   aag atg gtc gga ctg tcc gaa ggg tac gac cgg atc att gtc cgc ggc 1176
   Lys Met Val Gly Leu Ser Glu Gly Tyr Asp Arg Ile Ile Val Arg Gly
   340 345 350
   tct ttg gcg caa ccc gac ttc agc gtt ttc tac ctg cag gga gac cgg 1224
   Ser Leu Ala Gln Pro Asp Phe Ser Val Phe Tyr Leu Gln Gly Asp Arg
   355 360 365 370
   gta ttg gcg gtc gat aca gtg aac cgt cca gtg gag ttc aac cag tca 1272
   Val Leu Ala Val Asp Thr Val Asn Arg Pro Val Glu Phe Asn Gln Ser
   375 380 385
   aaa caa ata atc acg gat cgt ttg ccg gtt gaa cca aac cta ctc ggt 1320
   Lys Gln Ile Ile Thr Asp Arg Leu Pro Val Glu Pro Asn Leu Leu Gly
   390 395 400
   gac gaa agc gtg ccg tta aag gaa atc atc gcc gcc gcc aaa gct gaa 1368
   Asp Glu Ser Val Pro Leu Lys Glu Ile Ile Ala Ala Ala Lys Ala Glu
   405 410 415
   ctg agt agt gcc tgaaatctat acccacaata aatcaccgtt ttgccccata 1420
   Leu Ser Ser Ala
   420
   gcgtgtgagg ataaacag atg tct aaa gta gtg tat gtg tca cat gat 1468
   Met Ser Lys Val Val Tyr Val Ser His Asp
   425 430
   gga acg cgt cgc gaa ctg gat gtg gcg gat ggc gtc agc ctg atg cag 1516
   Gly Thr Arg Arg Glu Leu Asp Val Ala Asp Gly Val Ser Leu Met Gln
   435 440 445
   gct gca gtc tcc aat ggt atc tac gat att gtc ggt gat tgt ggc ggc 1564
   Ala Ala Val Ser Asn Gly Ile Tyr Asp Ile Val Gly Asp Cys Gly Gly
   450 455 460
   agc gcc agc tgt gcc acc tgc cat gtc tat gtg aac gaa gcg ttc acg 1612
   Ser Ala Ser Cys Ala Thr Cys His Val Tyr Val Asn Glu Ala Phe Thr
   465 470 475 480
   gac aag gtg ccc gcc gcc aac gag cgg gaa atc ggc atg ctg gag tgc 1660
   Asp Lys Val Pro Ala Ala Asn Glu Arg Glu Ile Gly Met Leu Glu Cys
   485 490 495
   gtc acg gcc gaa ctg aag ccg aac agc agg ctc tgc tgc cag atc atc 1708
   Val Thr Ala Glu Leu Lys Pro Asn Ser Arg Leu Cys Cys Gln Ile Ile
   500 505 510
   atg acg ccc gag ctg gat ggc atc gtg gtc gat gtt ccc gat agg caa 1756
   Met Thr Pro Glu Leu Asp Gly Ile Val Val Asp Val Pro Asp Arg Gln
   515 520 525
   tgg taaaccacaa tggtaaacca ctgcgagcca aaacagccga gcaggagcgc 1809
   Trp
   agtccggcaa caccttatta agcacatgcc gaaccctatt tgcagcgctt catgcctgca 1869
   aagtcccgat tgatgaaatc cgggctccaa gcaaggagcc cggaatctct caccgccacg 1929
   aaatcaatgg ccaatcccgg g 1950
<210> 17
   <211> 27
   <212> DNA
   <213> Artificial Sequence
<220>
   <223> STRANDNESS:single
<220>
   <223> TOPOLOGY:linear
<220>
   <223> Description of Artificial Sequence:SP-1
<400> 17
   tatgcgtcac tagtcgggag tgcgtta 27
<210> 18
   <211> 27
   <212> DNA
   <213> Artificial Sequence
<220>
   <223> STRANDNESS:single
<220>
   <223> TOPOLOGY:linear
<220>
   <223> Description of Artificial Sequence:SP-2
<400> 18
   tataacgcac tcccgactag tgacgca 27
<210> 19
   <211> 30
   <212> DNA
   <213> Artificial Sequence
<220>
   <223> STRANDNESS:single
<220>
   <223> TOPOLOGY:linear
<220>
   <223> Description of Artificial Sequence:CB-4F Primer
<400> 19
   gccccccata tgacagcttt gaatctgatg 30
<210> 20
   <211> 27
   <212> DNA
   <213> Artificial Sequence
<220>
   <223> STRANDNESS:single
<220>
   <223> TOPOLOGY:linear
<220>
   <223> Description of Artificial Sequence:CB-5R Primer
<400> 20
   gcactagtca gagacggacc ggcagac 27
<210> 21
   <211> 33
   <212> DNA
   <213> Artificial Sequence
<220>
   <223> STRANDNESS:single
<220>
   <223> TOPOLOGY:linear
<220>
   <223> Description of Artificial Sequence:154A1-1F
<400> 21
   gccccccata tggcgaccca gcagcccgcc ctc 33
<210> 22
   <211> 31
   <212> DNA
   <213> Artificial Sequence
<220>
   <223> STRANDNESS:single
<220>
   <223> TOPOLOGY:linear
<220>
   <223> Description of Artificial Sequence:154A1-2R Primer
<400> 22
   gcactagtca gccggcgtgc agcaggaccg g 31
<210> 23
   <211> 32
   <212> DNA
   <213> Artificial Sequence
<220>
   <223> STRANDNESS:single
<220>
   <223> TOPOLOGY:linear
<220>
   <223> Description of Artificial Sequence:DoxA-1F Primer
<400> 23
   gccccccata tggccgtcga cccgttcgcg tg 32
<210> 24
   <211> 31
   <212> DNA
   <213> Artificial Sequence
<220>
   <223> STRANDNESS:single
<220>
   <223> TOPOLOGY:linear
<220>
   <223> Description of Artificial Sequence:DoxA-2R Primer
<400> 24
   gcactagtca gcgcagccag acgggcagtt c 31

## Description

### Technical Field

This invention relates to a system for the expression of actinomycete cytochrome P-450 genes in *Escherichia coli.*

### Background Technology

Cytochrome P-450 enzymes (hereinafter referred to simply as "P-450s") which are encoded by cytochrome P-450 genes are a general name of a group of protoheme-containing proteins whose reduced form shows Soret band around 450 nm when bound to carbon monoxide. P-450s are bound to microsome in tissue of various kinds of animal or plant, or in fungi or yeasts, or to inner membrane of mitochondrion in tissue of some kind of animals. In certain kinds of bacteria and fungi, P-450s exist in soluble state.

P-450s show various types of substrate-specificity Some P-450s have abnormally so wide substrate-specificity that they can react with various kinds of organic compounds as substrate. Some, on the other hand, have considerably strict substrate-specificity, and react only with comparatively limited kinds of organic compounds. P-450s also show excellent stereo- and/or regio-specificity to reaction site. With regard to concrete functions, P-450s are known to catalyze reactions such as hydroxylation, epoxidation, dealkylation and denitrification, of xenobiotics in a cell which expresses said P-450s. For example, most of drugs which are administered to human are metabolized and inactivated in the body by various or specific action, such as hydroxylation, of P-450. In some cases, on the contrary, pharmacological effects of the drugs are improved, or subsidiary action is enhanced. P-450 is, therefore, medically very important from the viewpoint of the research of metabolism of medicine or the development of prodrugs.

Thus, P-450s which have drug-metabolizing functions for higher organisms including human have been studied from every angle for long years. Although these enzymes are obtained from microsome fractions of liver of higher organisms, it is difficult to purify these enzymes into single isozyme. On this account, there has been developed a technology to express functionally a gene which encodes single isozyme in a host such as *Escherichia coli* or yeast, and to thus conveniently investigate the metabolic role of the enzyme.

P-450s of higher organisms which have such drug-metabolizing functions as mentioned above have never been successfully applied to material production on industrial scale. P-450s of higher organisms, when functionally expressed in a host such as *Escherilchia coli* or yeast, show only low productivity as compared with P-450s of bacteria, and also cause various side reactions. For these reasons, P-450s of higher organisms have been used only restrictively.

In the case of P-450 originated from microorganisms such as fungi and bacteria, on the other hand, some kinds of P-450s are known to serve for the production of industrially useful materials. Some of such kinds of P-450s have actually been utilized for industrial production of useful medicine. Typical example is the production of pravastatin, a medicine to remedy hyperlipidemia, by means of the hydroxylation at 6β-position of compactin with *Storeptomyces carbophilus,* a species of actinomycete (Watanabe et al., Gene, 163 (1995) 81-85, Japanese Patent Application Laid-Open (Kokai) Publication No. Hei 6 (1994)-70780). There has also been put into practice a method by which to produce active vitamin D₃ by means of the hydroxylation at 1α- and 25-positions of vitamin D₃ with use of *Pseudonocardia autotrophica,* a species of actinomycete.

Such microbial conversion of drugs with use of actinomycetes cytochrome P-450 enzymes as mentioned above have been carried out with use of culture liquids or cells of actinomycetes which express said enzymes. There have also been used culture liquids wherein genes which encode actinomycete P-450s have been introduced into *Storeptomyces lividans* that is also a species of actinomycete and that is suitable as a host, to express the enzymatic activity. The microbial conversion of substrate compounds with the actinomycete strains which have such genes as mentioned above takes considerable time for the cultivation of the strains and for the conversion of substrate compounds into desired products. Furthermore, some enzymes need consideration on expression-inducible conditions under which to increase the amount of enzyme expressed. Moreover, some species of actinomycetes which are to be used for the conversion have, in themselves, a system for metabolizing or degrading substrate or desired product, which causes the formation of by-products or the reduction of substrate or desired product, and thus decreases the productivity of desired product.

There is also a report of experiment wherein, after the example of functionally expressing a gene which encodes single isozyme of the above-mentioned higher organism-originated P-450s in a host of microorganism such as *Escherichia coli,* CYP105D1 gene which is a *Storeptomyces griseus* originated cytochrome P-450 gene was functionally expressed in *Escherichia coli*(Taylor et al., Biochemical and Biophysical Research Communications (1999) 263: 838-842). It seems that, in this expression system, some suitable electron donor for the P-450 in periplasm of *Escherichia coli* hydroxylates hydrocarbons in cooperation with the P-450 (Kaderbhai et al, Applied and Environmental Microbiology, 67 (2001) 2136-2138).

Such a P-450 gene-expressing system has a merit that *Escherichia coli* as a host needs less time for cultivation as compared with actinomycetes or the like.

Taylor et al., 1999, disclose the expression of the cytochrome P450105D1 (CYP105D1) from Streptomyces griseus in E. coli. but mainly focus on the use of the thus obtained enzyme as a model for the study of the human drug metabolizing P450 (see page 842, left column, last sentence). In particular; the reactivity of said enzyme with various substances such as warfarin, α-benzoflavone, β-benzoflavone, 20-methylcholanthrene is shown.

JP-A-7 067 666 relates to a chimera P450 producing yeast strain simultaneously manifesting ferredoxin and ferredoxin reductase.

WO 93/12236 discloses the cloning of cytochrome P450 (soyC) and of a nucleotide sequence encoding a protein termed "ferredoxin-soy" from bacteria of the genus streptomyces. It aims at the provision of recombinant bacteria of the genus streptomyces capable of constitutive expression of cytochrome P450soy and the iron-sulfur protein (i.e., ferredoxin-soy, see page 3, lines 7 to 8) that donates electrons to cytochrome P450soy. However, it is completely silent on any related ferredoxin reductase gene and, consequently, also as regards its cloning or use in a corresponding expression system.

Peterson et al., Journal of Biological Chemistry, p. 6066-6073, 1990, report the cloning and sequencing of the open reading frames encoding putidaredoxin reductase and putidaredoxin, as well as the independent, i.e., separate expression of said enzymes in E. coli. Said proteins are thought to facilitate studies of the interaction with each other and with a specific enzyme designated P-450_{cam}, i.e., a cytochrome P450 protein from Pseudomonas putida. Said publication does not make any additional reference to a possible interaction of said putidaredoxin reductase and putidaredoxin enzymes with other cytochrome P450 proteins than P-450_{cam}.

Sibbesen et al., Journal of Biological Chemistry, p. 22462-22469, 1996, disclose a putidaredoxin reductase - putidaredoxin - cytochrome P450_{cam} triple fusion protein that constitutes a self-sufficient catalytic system for the oxidation of camphor and other substrates by P450_{cam}. The system is expressed in E. coli.

### Disclosure of Invention

The above-mentioned system for the conversion of organic compounds with microorganisms which have P-450s is intended to be used, for instance, for the application to biocatalyst or for the research of drug metabolism. In consideration of application to biocatalyst, in particular, more efficient bioconversion would be demanded. In order to achieve the efficient screening of industrially important and desired actinomycete P-450 enzymes, there would be needed a suitable gene library as an object of robotized enzyme-assaying operation or other convenient and rapid enzyme-assaying operation in high throughput screening or the like. Concretely, there is demanded a library which has actinomycete-originated different cytochrome P-450 genes (actinomycete cytochrome P-450-expression library) and wherein microorganism, preferably handy and quick-growing microorganism, is used as a host in which each constituent clone is capable of expression.

In order to attain the above-mentioned objective, it would be useful to use, as a host, *Escherichia coli* which, at least, needs comparatively short time for cultivation, and which is considered to have only a few systems for the metabolism or degradation of substrate compounds or products from said substrate compounds. It has been confirmed, however, that only introducing an actinomycete P-450 gene into a host *Escherichia coli* and cultivating the same, as in the above-mentioned Taylor *et al.* wherein *Escberichia coli* is used as a host, does not achieve the functional expression of most of various kinds of P-450 genes which are originated from other species of actinomycete (or, in other words, enzymatic activity of P-450 is not shown as expected). Thus, the inventors of the present invention have studied how to construct a system which is capable of functionally expressing actinomycete-originated various kinds of P-450 genes surely and with high enzymatic activity. As a result, they have found out that, when a certain electron transport system originated from microorganism which is xenogenic to *Escherichia coli* is introduced together with P-450 gene and is then allowed to co-express, the P-450 gene originated from various species of actinomycete is functionally expressed.

Based on such a finding as mentioned above, this invention provides a system for the expression of actinomycete cytochrome P-450 genes in host *Escherichia coli,* wherein said *Escherichia coli* supports a recombinant DNA molecule which comprises xenogenic microorganism-originated ferredoxin gene, ferredoxin reductase gene and said cytochrome P-450 gene in operable state.

Such an expression system as explained above is capable of the expression of such a gene as mentioned in the above Taylor *et al.* which encodes actinomycete cytochrome P-450, and which is incapable of conjugating native electron transport system of *Escherichia coli.* In other words, the expression system of this invention achieves expected enzymatic activity of P-450 whether P-450 gene may conjugate native electron transport system of *Escherichia coli* or not. Thus, the term "functionally express" in this specification means that a gene of interest expresses protein, which is encoded by the gene, in an active form.

In the following, this invention is explained in more detail.

Host *Escherichia coli* means certain kinds of *Escherichia coli* which are usable for the propagation of vectors such as plasmids and phages and of inserted genes. Any species of host will do if only, in a recombinant DNA experiment with use of host-vector system, a vector with an exogenous DNA fragment is capable of replication after transformation. As a host for said host-vector system, *Escherichia coli* on the market would be conveniently utilized.

Actinomycete cytochrome P-450 genes in this invention include P-450 genes originated from any genus of bacteria that belong to order *Actinomycetales* if only the bacteria have P-450 genes which serve to achieve the objective of this invention in some form or other (*e.g.,* on chromosome or plasmid). Thus, cytochrome P-450 genes include all that encodes protein mentioned above which has such activity as to catalyze single oxygen atom insertional reaction in accordance with this invention. Although not restrictive, examples of P-450 genes which are intended to be incorporated into-the expression system of this invention include those which have the above-mentioned function, at least a part of whose DNA sequences have been determined, and each of whose sequence information is available from gene data base (EMBL and GenBank), concretely, those which are originated from the following species of actinomycte and which encode protein having the above-mentioned activity, or those which have functions of cytochrome P-450 as mentioned below.

| Actinomycete | Function of cytochrome P-450 |
|---|---|
| *Amycolatopsis orientalis* | Unknown |
| *Actinomadura verrucosospora* | Biosynthesis of vercopeptin |
| *Amycolata autotrophica* | Unknown |
| *Amycolatopsis mediterranei* | Biosynthesis of rifamycin |
| *Amycolatopsis mediterranei* | Biosynthesis of balhimycin |
| *Kitasatospora griseospola* | Biosynthesis of terpentecin |
| *Mcromonospora griseorubida* | Biosynthesis of mycinamicin |
| *Micromonospora inyoensis* | Unknown |
| *Microtetraspora recticatena* | Hydroxylation of compactin |
| *Mycobacterium smegmatis* mc2155 | Degradation of piperidine and pyrrolidine |
| *Mycobacterium sp. FM10* | Unknown |
| *Mycobacterium tuberculosis* H37Rv | 22 P-450 genes in whole genome (function unknown) |
| *Myxococcus xanthus* | Polyketide antibiotic TA |
| *Pseudonocardia autotrophica* | Hydroxylation of vitamin D₃ |
| (old name: *Amycolata autotrophica*) | |
| *Rhodoococcus erythropolis* | Degradation of thiocarbamate herbicide |
| *Rhodococcus fascians* (D188) | Synthesis of phytophysiologically active substance |
| *Rhodococcus ruber* | Degradation of ethyl- *tert*-butyl ether |
| *Saccharopolyspora erythraea* | Hydroxylation of erythromycin |
| *Streptoalloteichus hindustanus* | Unknown |
| *Streptomyces acidiscabies* | Biosynthesis of thaxtomin A |
| *Streptomyces albus* | Unknown |
| *Streptomyces ansochromogenes* | Biosynthesis of nikkomycin |
| *Streptomyces antibioticus* | Biosynthesis of oleandomycin |
| *Streptomyces antibioticus* | Biosynthesis of simocyclinone |
| *Streptomyces aureofaciens* Ren71 | Unknown |
| *Streptomyces avermitilis* | Formation of furan ring of avermectin |
| *Streptomyces avermitilis* | Biosynthesis of oligomycin |
| *Streptomyces avermitilis* | Biosynthesis of polyketide-4 |
| *Streptomyces avermitilis* | Biosynthesis of polyketide-9 |
| *Streptomyces avermitilis* | Biosynthesis of other type polyketide |
| *Streptomyces avermitilis* | Biosynthesis of polyene macrolide |
| *Streptomyces avermitilis* | Biosynthesis of peptide-7 |
| *Streptomyces carbophilus* | Hydroxylation of compactin |
| *Streptomyces clavuligerus* | Biosynthesis of clavulanic acid |
| *Streptomyces coelicolor A3(2)* | 18 P-450 genes in whole genome |
| | (function unknown) |
| *Streptomyces fluvus* | Hydroxylation of compactin |
| *Streptomyces fradiae* | -Biosynthesis of tylosin |
| *Streptomyces glaucescens* | Unknown |
| *Streptomyces griseolus* | Degradation of sulfonylurea herbicide |
| *Streptomyces griseus* | Unknown |
| *Streptomyces hygroscopicus* | Biosynthesis of rapamycin |
| *Streptomyces hygroscopicus* | Biosynthesis of FK520 |
| var. *ascomyceticus* | |
| *Streptomyces lavendulae* | Biosynthesis of mitomycin |
| *Streptomyces lavendulae* | Biosynthesis of complestatin |
| *Streptomyces lividans* | Unknown |
| *Streptomyces maritimus* | Biosynthesis of enterocin |
| *Streptomyces natalensis* | Biosynthesis of pimaricin |
| *Streptomyces nodosus* | Biosynthesis of amphotericin |
| *Streptomyces nogalater* | Biosynthesis of nogalamycin |
| *Streptomyces noursei* | Biosynthesis of nystatin |
| *Streptomyces peucetius* | Hydroxylation of daunomycin |
| *Streptomyces peucetius* | Hydroxylation of daunomycin |
| *subsp. caesius* | |
| *Streptomyces rishiriensis* | Biosynthesis of coumermycin |
| strain DSM 40489 | A1 |
| *Streptomyces sclerotialus* | Unknown |
| *Streptomyces* sp*.* | Hydroxylation of FK-506 |
| *Streptomyces* sp. | Unknown |
| *Streptomyces spheroids* | Biosynthesis of novobiocin |
| *Streptomyces tendae* | Biosynthesis of nikkomycin |
| *Streptomyces thermotolerans* | Epoxidation of carbomycin |
| *Streptomyces venezuelae* | Biosynthesis of pikromycin, methymycin |

the following actinomycete P-450 are also included as usable in this invention. Each of the following literatures gives guidance how to prepare gene which encodes each enzyme.
Compactin-hydroxylating enzyme originated from *Streptomyces*

| | |
|---|---|
| *carbophilus* (P-450_{aca}-2): | Watanabe et al., Gene 163 (1995) 81-85 or Japanese Laid-Open (Kokai) Patent Publication No. Hei 6 (1994)-70780 |
| *Microtetraspora recticatena:* | Japanese Laid-Open (Kokai) Patent Publication No. 2001-286293 |
| Vitamin D₃-hydroxylating | enzyme originated from *Amycolata* sp.: Sasaki et al., Applied Microbiology and Biotechnology (1992) 38: 152-157 |

*Streptomyces roseochromogenes-*originated progesterone-hydroxylating enzyme (Berrie et al., Journal of Steroid Biochemistry & Molecular Biology 77 (2001) 87-96) can also be mentioned. Although gene sequence of this *Streptomyces roseochromogenes* is not mentioned in published literatures, function and biochemical properties of this P-450 enzyme have detailedly determined, and, on the basis of which information, it is easy to prepare gene which encodes said P-450 enzyme.

Cytochrome P-450-encoding genes (or P-450 genes) as mentioned in this invention include any gene so long as it can be isolated from total DNAs of the above-mentioned actinomycetes or can be amplified by PCR reaction, which is mentioned later, on the basis of information of nucleotide sequences of said total DNAs, and so long as it is capable of functional expression in the system of this invention for the expression of P-450 genes.

The actinomycete cytochrome P-450 gene may be the compactin-hydroxylating enzyme-encoding gene (*moxA*) originated from *Microtetraspora recticatena.* Also included in P-450 genes of this invention are polynucleotides which are functionally equivalent to the above-mentioned genes (also called native gene), and which have activity to catalyze single oxygen atom insertional reaction against corresponding substrates in the expression system of this invention. It is guessed that complement base sequences of said equivalent polynucleotides hybridize with corresponding native genes under a certain hybridization condition, *e.g*., under stringent condition in 2 × SSC (standard saline citrate) at 60°C, preferably in 0.5 × SSC at 60°C, most desirably 0.2 × SSC at 60°C. When each of the polynucleotides is lined up side by side with the corresponding native gene, there would be shown homology of 80 %, preferably 90 %, most desirably at least about 95 %. This "% homology" means percentage of nucleotide which is in common between two sequences when the two sequences are lined up side by side with each other in an optimum manner. [Thus, "% homology" = (number of coincident positions/total number of positions) × 100. This can be calculated with use of algorithm on the market. Such an algorithm is incorporated in NBLAST and XBLAST programs which are mentioned in Altschul et al., J. Mol. Biol. 215 (1990) 403-410.] Specifically, a functionally equivalent polynucleotide of a P-450 gene has a homology of at least 80% to said nucleotide sequence and has the activity to catalyze single oxygen atom insertional reaction against corresponding substrates in the expression system of this invention.

Ferredoxin gene which is incorporated in the expression system of this invention is a DNA molecule which is originated from microorganisms (or bacteria) which are xenogenic to host *Escherichia coli.* Ferredoxin gene generally encodes protein which functions as an electron transporter having a molecular weight of about 6,000 to 14,000. Ferredoxin gene may be originated from bacteria except *Escherichia coli* so long as the bacteria participate in the functional expression of P-450 gene when co-expressed with the above-mentioned actinomycete P-450 gene and further with ferredoxin reductase gene which will be mentioned later. Concrete examples of said bacteria include actinomycete which may or may not be the same as mentioned above from which P-450 gene is originated.

Also usable is ferredoxin gene originated from bacteria, *e.g.*, of genus *Pseudomonas,* which belong to different genus from that of actinomycete from which P-450 gene is originated. Such a ferredoxin gene is putidaredoxin gene (also called *camB*) as mentioned in Peterson et al., The Journal of Biological Chemistry, 265 (1990) 6066-6073.

When ferredoxin gene is originated from the same actinomycete from which P-450 gene is originated, P-450 gene and ferredoxin gene mary sometimes constitute a gene cluster in which said P-450 gene and ferredoxin gene exist adjacent to each other on genomic DNA. In such a case, a DNA fragment which contains both of said genes may be used in the expression system of this invention. In the expression system of this invention, ferredoxin gene may exist with another ferredoxin. The specific example of such a case is the use of ferredoxin gene originated from actinomycete in combination with the above-mentioned camB originated from *Pseudomonas putida.* Such a ferredoxin gene also includes functionally equivalent polynucleotide which can be specified in the same manner as in the above-mentioned P-450 gene.
In the present invention, the ferredoxin gene is the putidaredoxin gene (*camB*) originated from *Pseudomonas putida,* or the ferredoxin gene (*moxB*) of *Microtetraspora recticatena,* and/or it originates from one and the same gene cluster of actinomycete as the cytochrome P-450 gene.

Ferredoxin reductase gene which is incorporated in the expression system of this invention as an essential factor may be originated from bacteria which are xenogenic to host *Escherichia coli,* and which, under circumstances, may also be xenogenic to the origin of P-450 gene. Concretely, ferredoxin reductase gene originated from any bacteria is usable in this invention so long as the bacteria are capable of co-expression with the above-mentioned P-450 gene and with ferredoxin gene, and so long as the gene encodes ferredoxin reductase which shows the expected activity of P-450 enzyme, i.e., the product of said gene expression of P-450, or, in other words, which catalyzes single oxygen atom insertional reaction against substrate. Such ferredoxin reductase genes are the ferredoxin reductase gene originated from *Streptomyces coelicolor* (hereinafter sometimes referred to as "fdr-1" or "fdr-2") and putidaredoxin reductase gene originated from the above-mentioned *Pseudomonas putida* (hereinafter referred to also as *camA*). Such a gene also includes functionally equivalent polynucleotide which can be specified in the same manner as in the above-mentioned P-450 gene.

In the expression system of this invention, the above-mentioned . P-450 gene, ferredoxin gene and ferredoxin reductase gene are introduced in *Escherichia coli* in an operable state. The term "operable state" means that said genes are present in host in such a manner that all of the genes are capable of functional expression. In a typical example of such a state, all of the above-mentioned genes exist in a plasmid, which is capable of autonomous replication in *Escherichra coli,* together with autonomously replicating sequence, promoter sequence, terminator sequence and drug resistant gene, in a suitable order. Otherwise, all of said genes exist in chromosome of host *Escherichia coli* in such a manner that they are capable of functional expression via a chromosomal DNA integrative vector. The above-mentioned P-450 gene, ferredoxin gene and ferredoxin reductase gene may be arranged in any order in said plasmid. Usually, however, P-450 gene is preferably placed uppermost in the stream. When, in particular, P-450 gene and ferredoxin gene are used as a gene cluster fragment of the same origin, the following orders may be preferable: P-450 gene-ferredoxin gene-ferredoxin reductase gene; P-450 gene-ferredoxin gene-putidaredoxin reductase gene-putidaredoxin gene; or P-450 gene-putidaredoxin reductase gene-putidaredoxin gene.

Plasmid or vector which is usable in the above-mentioned expression system may be capable of stable autonomous replication in *Escherichia coli,* or may be an integrative vector which is capable of integrating chromosome of *Escherichia coli* with exogenous gene. Both can be available from those on the market, or by modification where necessary. Plasmids which have a strong promoter for gene transcription are conveniently used for the above-mentioned purpose. Examples of such plasmids include those on the market, such as pET11 and pUC18.

Thus provided actinomycete P-450 gene expression system of this invention is usable for the screening of P-450 enzymes which are suitable for the modification of various kinds of drugs or the bioconversion from precursor into desired drugs, or further for the manufacture of desired drugs from precursor.

### Brief Explanation of Drawings

Fig. 1 shows the structure of plasmid pMoxAB.
Fig. 2 shows the structure of plasmid pMoxAB-fdr1.
Fig. 3 shows the structure of plasmid pMoxAB-fdr2.
Figs. 4 shows the structure of plasmid pMoxAB-camAB.
Fig. 5 shows the structure of plasmid pT7NS-camAB.
Fig. 6 shows the structure of plasmid pCBM-camAB.
Fig. 7 shows the structure of plasmid pSC154A1-camAB.
Fig. 8 shows the structure of plasmid pDoxA1-camAB.

In the following, this invention is concretely explained with working examples.

### Best Mode for Working this Invention

This invention will be further explained with reference to examples of the construction of P-450 gene which forms pravastatin of the following formula: 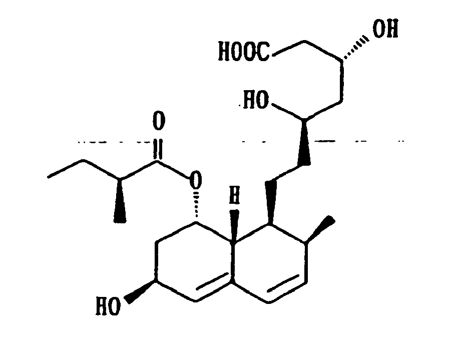 or a mixture (which is called "RT-5.8 substances" in this specification) of isomers thereof which have the following formulae: 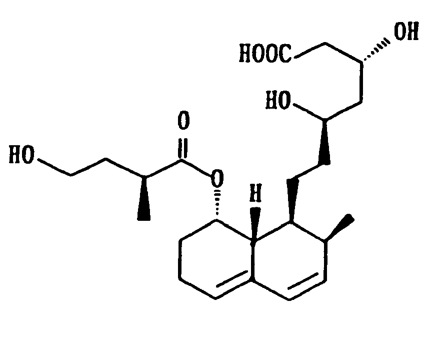 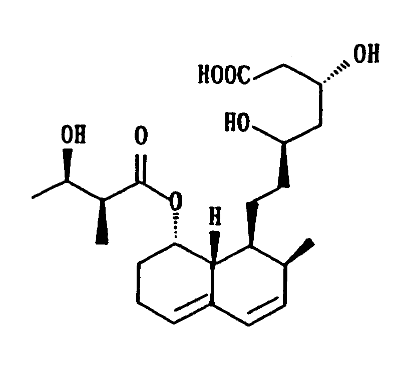 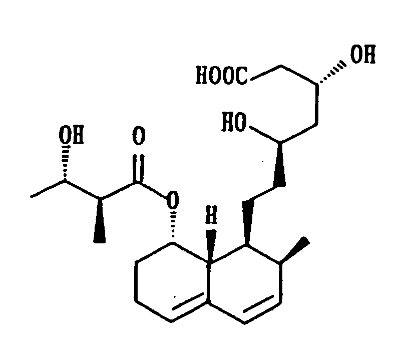 by means of single oxygen atom insertional reaction of compactin of the following formula: 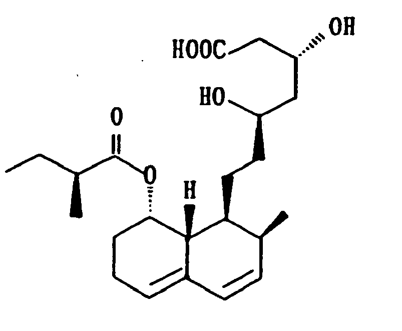 (or also existent as δ-lactone compound corresponding to the above formula) or a salt thereof.

Incidentally, pravastatin sodium is a clinically important medicine as an agent to cure hyperlipidemia.

Actinomycete which has an enzymatic activity to hydroxylate compactin (also called mevastatin) belongs to genus *Streptomyces* (Japanese Laid-Open (Kokai) Patent Publication Sho 57 (1982)-50894, Japanese Patent No. 2672551) or to genus *Microtetraspora*. As for the latter, which has been confirmed by the inventors of this invention, a DNA fragment which contains P-450 gene can be prepared by the following process.

### Preparation of P-450 gene from Microtetraspora recticatena IFO 14525

Said gene can be obtained by polymerase chain reaction (PCR) with use of primers which have been designed in accordance with an amino acid sequence of the region which is known to keep amino acid sequence with a high probability among a family of lot of P-450 hydroxylation enzymes (J. Bacteriol. 172, 3335-3345 (1990)). For example, IFO 14525 strain is cultivated under certain cultivation conditions, and, then, thus obtained cells are crushed to give chromosomal DNA. Thus obtained chromosomal DNA is subjected to PCR reaction with use of primers which have been designed from amino acid sequences for oxygen-binding domain and heme-binding domain which exist in common with P-450 hydroxylation enzyme family. There is obtained a DNA fragment which has been amplified by the PCR reaction, on the basis of which a further PCR reaction is conducted, and, thus, there is obtained flanking regions of the DNA fragment which has been amplified by the first PCR reaction (in the downstream, there existed a gene which encoded ferredoxin). All of the above-mentioned manipulation can be carried out by any method that is known well in this art. Details of these sets of manipulation are mentioned in the specification of Japanese Patent Application No. 2001-47664 by the same applicant as that of the present application.

Sequence No. 1 in the sequence listing shows a nucleotide sequence (and amino acid sequences encoded) which includes adjacent region of thus obtained P-450 gene.

In the above-mentioned sequence, a continuous nucleotide sequence from base 313 to base 1533 corresponds to P-450 gene (*mox*A), and a continuous nucleotide sequence from base 1547 to base 1741 corresponds to ferredoxin gene (*mox*B).

### Preparation of P-450 gene from Streptomyces sp. TM-6 or TM-7

From among a lot of microorganisms that belong to *Streptomyces* which were isolated from the soil in Japan, the applicant has identified the above-captioned TM-6 and TM-7 strains as microorganisms which are capable of biologically converting compactin as a substrate into pravastatin. Said strains were deposited on April 25, 2001, at the International Patent Organism Depository (IPOD) in the National Institute of Advanced Industrial Science and Technology (AIST) at Tsukuba Central 6, 1-1-1 Higashi, Tsukuba, Ibaraki, Japan, and thus have been received with Deposit Nos. FERM P-18311 and FERM P-18312.

Later, a demand was made on the above-mentioned IPOD, which is also an international depositary authority under the Budapest Treaty, for the transfer of these strains TM-6 and TM-7 to said international depositary authority under said Treaty, and, thus, these strains have been received with Deposit Nos. FERM BP-8002 and FERM BP-8003.

With regard to said TM-7 strain, a region of target gene was amplified by PCR in the same manner as in the above-mentioned IFO 14525, and, thus, the sequence of DNA fragment containing the target gene and its adjacent region was determined. The result is shown in Sequence No. 2. In this sequence, a continuous nucleotide sequence from base 544 to base 1758 corresponds to P-450 gene (*box*A)*,* and a continuous nucleotide sequence from base 1782 to base 1970 corresponds to ferredoxin gene (*boxB*). The manipulation to obtain these genes is mentioned detailedly in the specification of Japanese Patent Application No. 2001-166412 which has been filed by the applicant of the present application.

When compared with the sequence of P-450 gene of *Streptomyces carbophilus SANK* 62585 strain (FERM BP-1145) which is mentioned, for instance, in Japanese Patent No. 2672551, the nucleotide sequence of the above-mentioned *box*A was found to have a homology of about 75 %. With the above-mentioned *mox*A*,* the *box*A has a homology of about 46 %. With hydroxylation enzyme gene of *Streptomyces Lividans* the *box*A has a homology of about 75 %. Said *box*A has a homology of about 46 % with a gene encoding pyridylhomothreonine monooxygenase which is an intermediate in the course of biosynthesis of nikkomycin by *Streptomyces tendae* Tji 901 strain.

On the basis of the above explanation or explanation in working examples mentioned below or, furthermore, on the basis of techniques which are known well in this art or of information in gene database, anyone skilled in the art would be able to obtain various kind of P-450 genes by means of firstly screening actinomycetes which are known (from type culture catalogue published by ATCC) with respect to bio-conversion of substrates for single oxygen atom insertion, then identifying strains having expected enzumatic activity, and thus conducting PCR operation as mentioned above. Hence, actinomycete cytochrome P-450 genes as called in this invention include not only known ones but also all that skilled persons could obtain.

The preparation of ferredoxin gene and ferredoxin reductase gene which are included in the expression system of this invention, and the construction of expression system by means of operable connection between these genes and P-450 genes, could be achieved quite easily by anyone skilled in the art in the same manner as in the above-mentioned P-450 genes, or in accordance with methods as mentioned in literatures (Sambrook et al., Molecular Cloning, A Laboratory Manual, 3rd edition (2001), Cold Spring Harbor Laboratory Press, New York), and in the light of the methods in working examples as mentioned later.

Thus constructed expression system for P-450 genes is capable of functionally expressing P-450 genes under conditions where *Escherichia coli* is grown. When such an expression system is incubated under a suitable condition together with a substrate for enzyme as a product of P-450 gene (or when transformant as an expression system is cultivated), there is obtained a product wherein single oxygen atom has been inserted in substrate.

Cultivation is usually conducted on a medium which can be a nutritious medium for *Escherichia coli,* and which has no adverse effects on biological conversion of substrate. Such a medium is composed of suitable carbon source, nitrogen source, inorganic salt and natural organic nutriment. As said carbon source, there can be used glucose, fructose, glycerol, sorbitol and organic acids, either singly or in combination. The concentration of these carbon sources when used is suitably about 1 to 10 %, not particularly limited. As said nitrogen source, there can be employed one or two from ammonia, urea, ammonium sulfate, ammonium nitrate and ammonium acetate. As said inorganic salt, there can be used salts such as potassium dihydrogenphosphate, dipotassium hydrogenphosphate, magnesium sulfate, manganese-sulfate and ferrous sulfate. As organic nutrient which has growth promoting effects on microorganism used, there can be used peptone, meat extract, yeast extract, corn steep liquor and casamino acids. Furthremore, a small amount of vitamins and nucleic acids may be included in medium.

In the expression system of this invention, high-titer P-450 enzymes with expected activity can be obtained when P-450 enzymes are induced at a temperature of about 25°C or less, preferably at 20 to 24°C, after host *Escherichia coli* has been cultivated at a temperature suitable for the growth of *Escherichia coli, e.g*. at 28 to 40°C.

The following is a detailed explanation of an example of construction of expression system for *mox*A gene originated from *Microtetraspora recticatena* IFO 14525 which encodes a compactin-hydroxylating enzyme as an instance of actinomycete cytochrome P-450 enzymes. This invention is, however, not restricted at all by this example.

### Polymerase Chain Reaction (PCR):

In the following example, PCT is conducted under conditions as follows.

| (1) Condition where genomic DNA is used as a template: | |
|---|---|
| (Composition of reaction liquid) | |
| Sterilized purified water | 15 µl |
| Twice-concentrated GC buffer I (Takara Shuzo) | 25 µl |
| dNTP mixed solution (dATP, dGTP, dTTP, dCTP each 2.5 mM) | 8 µl |
| Forward primer (100 pmol/µl) | 0.5 µl |
| Reverse primer (100 pmol/µl) | 0.5 µl |
| Genomic DNA (10 ng/µl) | 0.5 µl |
| LA Taq (5 units/µl Takara Shuzo) | 0.5 µl |
| (Temperature condition) | |
| 94°C 3 minutes | |
| (98°C 20 seconds; 63°C 30 seconds; 68°C 2 minutes) 30 cycles | |
| 72°C 5 minutes | |
| (2) Condition where plasmid DNA (pMoxAB-fdr1 | |
| is used as a template: | |
| (Composition of reaction liquid) | |
| Sterilized purified water | 15 µl |
| Twice-concentrated GC buffer I (Takara Shuzo) | 25 µl |
| dNTP mixed solution (dATP, dGTP, dTTP, dCTP each 2.5 mM) | 8 µl |
| Mox-3F primer (100 pmol/µl) | 0.5 µl |
| Mox-5R primer (100 pmol/µl | 0.5 µl |
| Plasmid DNA (1 ng/µl | 0.5 µl |
| LA Taq (5 units/µl Takara Shuzo) | 0.5 µl |
| (Temperature condition) | |
| 94°C 3 minutes | |
| (98°C 20 seconds; 63°C 30 seconds; 68°C 2 minutes) 25 cycles | |
| 72°C 5 minutes | |

### Example 1 Construction of plasmid

### (1) pT7-fdr1

PCR was carried out with use of primer FDR1-1F
(5'-GCCATATGACTAGTGCGCCTCACAGACTGGAACGGGAATCTCATG -3') (see Sequence No. 3) and FDR1-2R
(5'-GCGAATTCTGTCGGTCAGGCCTGGTCTCCCGTCGGCCG-3') (see Sequence No. 4) by using, as a template, genomic DNA of *Streptomyces coelicolor*A3(2) [imparted by John Innes Institute (Norwich, UK)], and, thus, there was amplified a 1.3-kb fragment of gene (hereinafter referred to as *fdr-1;* see Sequence No. 5) encoding a protein which has homology with ferredoxin reductases. This fragment was treated with restriction enzyme Nde I and Bam HI, and was then subjected to electrophoresis in 0.8 % agarose gel. After the electrophoresis was over, the *fdr-1* gene fragment was recovered, with use of SUPREC-01 (Takara Shuzo), from a gel piece containing said gene fragment, which had been cut out from the gel, and was purified. Said fragment was ligated to *Nde* I site and *Bam* HI site of *Escherichia coli* plasmid vector pET11a (manufactured by Stratagene Co.) with use of T4 DNA ligase, and, then, *Escherichia coli* DH5α was transformed with the resultant DNA reaction liquid, and, thus, pT7-fdr1 was constructed.

### (2) pT7-fdr2

Under the same condition, PCR was carried out with use of primer FDR2-3F
(5'-CGACTAGTGACGAGGAGGCAGACAAATGGTCGACGCGGATCAG-3 ') (see Sequence No. 6) and FDR2-4R
(5'-CGGGATCCGACAACTATGCGACGAGGCTTTCGAGGG-3') (see Sequence No. 7) by using genomic DNA of the above-mentioned *Streptomyces coelicolor*A3(2), and, thus, there was amplified a 1.3-kb fragment of gene (hereinafter referred to as *fdr-2,* see Sequence No. 8), which is different from *fdr-1,* encoding a protein which has homology with ferredoxin reductases. This fragment was treated with restriction enzyme *Bam* HI and *Spe* I, and was then subjected to electrophoresis in 0.8 % agarose gel. After the electrophoresis was over, *fdr-2* gene fragment was recovered, with use of SUPREC-01 (Takara Shuzo), from a gel piece containing said gene fragment, which had been cut out from the gel, and was purified. Apart from that, plasmid pT7-fdr1 was treated with *Bam* HI and *Spe* I*,* and was then subjected to electrophoresis in 0.8 % agarose gel. After the electrophoresis was over, pET11a vector fragment was recovered, with use of SUPREC-01 (Takara Shuzo), from a gel piece containing said vector fragment which had been cut out from the gel, and was purified. Said vector fragment and the above-mentioned *fdr-2* gene fragment were ligated to each other with use of T4 DNA ligase, and, then, *Escherichia coli* DH5α was transformed, and, thus, pT7-fdr2 was constructed.

### (3) pT7-camAB

PCR was carried out with use of primer PRR-1F
(5'-GCCCCCCATATGAACGCAAACGACAACGTGGTCATC-3') (see Sequence No. 9) and PRR-2R
(5'-GCGGATCCTCAGGCACTACTCAGTTCAGCTTTGGC-3') (see Sequence No. 10) by using, as a template, genomic DNA of *Pseudomonas putida* ATCC17453, and, thus, there was amplified a 1.65 kb fragment (camAB fragment; see Sequence No. 16) which contained putidaredoxin reductase gene (*camA*) and putidaredoxin gene (*camB*) which was just downstream of said *camA.* This fragment was treated with restriction enzyme *Nde* I and *Bam* HI, and was then subjected to electrophoresis in 0.8 % agarose gel. After the electrophoresis was over, *camAB* fragment was recovered, with use of SUPREC-01 (Takara Shuzo), from a gel piece containing said fragment, which had been cut out from the gel, and was purified. Said fragment was ligated to *Nde* I site and *Bam* HI site of *Escherichia coli* plasmid vector pET11a (manufactured by Stratagene Co.) with use of T4 DNA ligase, and, then, *Escherichia coli* DH5α was transformed, and, thus, pT7-camAB was constructed.

### (4) pMoxAB

PCR was carried out with use of primer Mox-1F
(5'-GCCCCCCATATGACGAAGAACGTCGCCGACGAACTG-3') (see Sequence No. 11) and Mox-12R
(5'-GCAGATCTAGTGGCTTCAGGCGTCCCGCAGGATGG-3') (see Sequence No. 12) by using, as a template, genomic DNA of IFO14525 strain, and, thus, there was amplified a 1.4-kb fragment (*mox*AB fragment) which contained a gene (*mox*A) encoding compactin-hydroxylation enzyme and ferredoxin gene (*mox*B) which was adjacent downstream thereto. This fragment was treated with restriction enzyme *Nde* I and *Bgl*II I, and was then subjected to electrophoresis in 0.8 % agarose gel. After the electrophoresis was over, moxAB fragment was recovered, with use of SUPREC-01 (Takara Shuzo), from a gel piece containing said fragment, which had been cut out from the gel, and was purified. Said fragment was ligated to *Nde* I site and *Bam* HI site of the above-mentioned plasmid pET11a with use of T4 DNA ligase, and, then, *Escherichia coli* DH5α was transformed with resultant reaction liquid, and, thus, plasmid pMoxAB was constructed.

### (5) pMoxAB-fdr1 and pMoxAB-fdr2

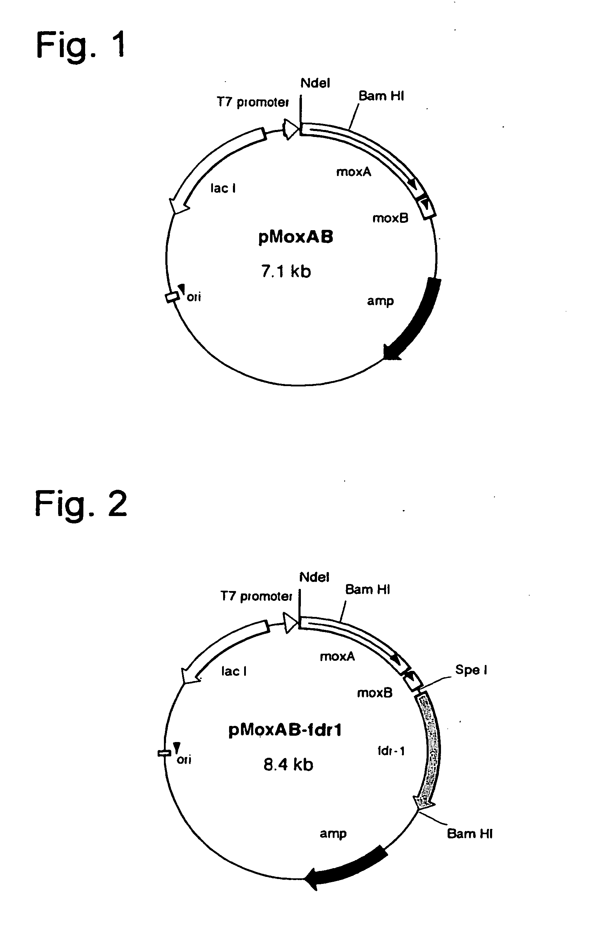

PCR was carried out with use of primer Mox-1F
(5'-GCCCCCCATATGACGAAGAACGTCGCCGACGAACTG-3') (see Sequence No. 11 as mentioned above) and Mox-2R
(5'-CGACTAGTGGCTTCAGGCGTCCCGCAGGATGG-3') (see Sequence No. 13) by using, as a template, genomic DNA of IFO14525 strain, and, thus, there was amplified a 1.4-kb fragment (*mox*AB fragment) which contained a gene (*mox*A) encoding compactin-hydroxylation enzyme and ferredoxin gene (*mox*B) which was adjacent downstream thereto. This fragment was treated with restriction enzyme *Nde* I and *Spe* I, and was then subjected to electrophoresis in 0.8 % agarose gel. After the electrophoresis was over, *mox*AB fragment was recovered, with use of SUPREC-01 (Takara Shuzo), from a gel piece containing said fragment, which had been cut out from the gel, and was purified. Said fragment was ligated to *Nde* I site and *Spe* I site of the above-mentioned plasmid pT7-fdr1 with use of T4 DNA ligase, and, then, *Escherichia coli* DH5α was transformed with resultant reaction liquid, and, thus, plasmid pMoxAB-fdr1 was constructed. Fig. 2 shows the structure of this pMoxAB-fdr1.

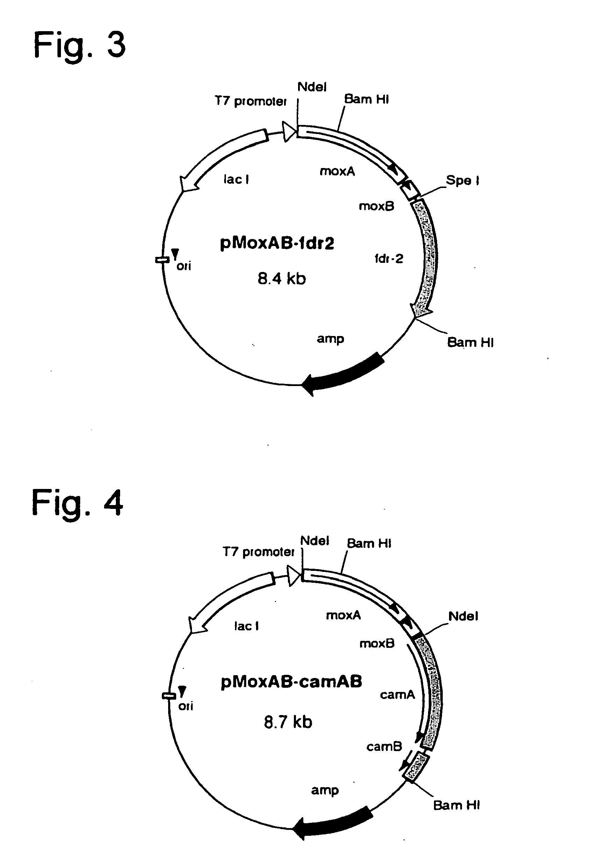

The same inserted fragment was ligated to *Nde* I site and *Spe* I site of another plasmid pT7-fdr2 with use of T4 DNA ligase, and, then, *Escherichia coli* DH5α was transformed with resultant reaction liquid, and, thus; plasmid pMoxAB-fdr2 was constructed. Fig. 3 shows the structure of this pMoxAB-fdr2.

### (6) pMoxAB-camAB

PCR was carried out with use of primer Mox-3F
(5'-GGAGATATACATATGACGAAGAAC-3') (see Sequence No. 14) and Mox-5R
(5'-GCCCCCCATATGACGCACTCCTAGTGGCTTCAGGCGTCCCG-3') (see Sequence No. 15) by using, as a template, DNA of pMoxAB-fdr1, and, thus, there was amplified a 1.5-kb fragment which contained a gene encoding cytochrome P-450 enzyme having compactin-hydroxylating activity and ferredoxin gene (*mox*AB) which was adjacent downstream thereto. This fragment was ligated to *Nde* I site of the plasmid pT7-camAB with use of T4 DNA ligase, and, then, *Escherichia coli* DH5α was transformed with resultant reaction liquid, and, thus, plasmid pMoxAB-camAB was constructed. Fig. 4 shows the structure of this pMoxAB-camAB.

### Example 2 Preparation of recombinant which has actinomycete cytochrome P-450 enzymatic activity

*Escherichia coli* BL21(DE3) was transformed with three plasmids, *i.e.,* pMoxAB-fdr1, pMoxAB-fdr2 and pMoxAB-camAB, and, thus, transformant strains corresponding to these plasmids were obtained. Single colony of each of these strains was seeded on 2 ml of LB medium, and was subjected to shake culture at 28°C for 16 hours at 220 rpm. Thus obtained culture liquid in an amount of 200 µl was mixed with an equal amount (200 µl) of 40 % glycerol (sterilized) to give a glycerol culture, which was preserved at -80°C until used. On the other hand, with use of pMoxAB and pET11a which was used as a vector, *Escherichia coli* BL21(DE3) was transformed, and, thus, transformant strains corresponding to these plasmids were obtained. Said transformant strains were used as control.

### Example 3 Production of pravastatin and its hydroxylated analogues from compactin

### (1) Production process with use of static cells:

Glycerol culture of transformant strain of BL21(DE3) as obtained in the above Example 2 in an amount of 10 µl was added to 2 ml of LB medium to which 50 µg/ml (final concentration) of ampicillin had been added, and was then subjected to shake culture at 28°C for 16 hours at 220 rpm. The resultant culture liquid in an amount of 250 µl was added to 25 ml of NZCYM medium to which 50 µg/ml (final concentration) of ampicillin had been added, and was then subjected to shake culture at 37°C for 2.5 hours. Then, 25 µl of 100 mM IPTG and 25 µl of 80 mg/ml 5-aminolevulinic acid were added in this order, and the resultant mixture was subjected to shake culture at 18-28°C (this temperature is hereinafter called as "induction temperature") for 16 hours at 120 rpm. Cells were recovered by centrifugation from 10 ml of the resultant culture liquid, and were then washed once with conversion buffer2 (50 mM NaH₂PO₄, 1 mM EDTA, 0.2 mM DTT, 10 % glycerol, [pH 7.3]). Subsequently, the cells were suspended in 1 ml of said buffer to give a suspension of static cells. To this suspension of static cells, there were added compactin sodium salt (final concentration 250 µg/ml) and NADPH (final concentration 1 mM), and the resultant mixture was incubated at 32°C for 24-48 hours (this time is hereinafter referred to as "conversion time") under shaking condition (220 rpm). Later, to 100 µl of thus obtained reaction liquid, there was added 100 µl of acetonitrile, and the resultant mixture was subjected to vortex for one minute at room temperature, and was then centrifuged for 10 minutes at 16,000 rpm with an Epfendorf centrifugator. So obtained supernatant was analyzed with HPLC, and, thus, there were detected pravastatin and other hydroxylated analogues which had been formed by the hydroxylation of substrate compactin. The following shows detailed condition for this HPLC.

| | | |
|---|---|---|
| Analytical apparatus: | Shimadzu C-4RA Chromatopac | |
| Column: J' sphere ODS-H80 (YMC, Inc.), 75 mm × 4.6 mm I.D. | | |
| Mobile phase A: Ion-exchange water/acetic acid/triethylamine = 998:1:1 | | |
| B: Methanol/acetic acid/triethylamine = 998:1:1 | | |
| | | |
| Gradient time program: | 0 minute Mobile phase A/B = 50:50 | |
| | 3.00 minute Mobile phase A/B =10:90 | |
| | 3.50 minute Mobile phase A/B = 10:90 | |
| | 3.51 minute Mobile phase A/B = 50:50 | |
| | 6.00 minute End | |
| | | |
| Flow rate: 2.0 ml/minute | | |
| Detection: UV 237 nm | | |
| Injection content: 10 µl | | |
| Column temperature: 40°C | | |
| Analysis time: 6 minutes | | |
| Retention time: compactin | | 4.2 minutes |
| pravastatin | | 2.7 minutes |
| RT5.8 substances | | 3.6 minutes |

### (2) Production process by Fed-batch method:

Glycerol culture of transformant strain of BL21(DE3) in an amount of 10 µl was added to 2 ml of LB medium to which 50 µg/ml (final concentration) of ampicillin had been added, and was then subjected to shake culture at 28°C for 16 hours at 220 rpm. The resultant culture liquid in an amount of 250 µl was added to 25 ml of M9-plus medium (M9 salt, 0.4 % glucose, 0.5 % casamino acids, 100 µg/ml thiamin, 20 µl/ml thymine, 0.1 mM CaCl₂, 1 mM MgCl₂) to which 50 µg/ml (final concentration) of ampicillin had been added, and was then subjected to shake culture at 37°C for 2.5 hours. Then, 25 µl of 100 mM IPTG and 25 µl of 80 mg/ml 5-aminolevulinic acid were added in this order, and the resultant mixture was subjected to shake culture at 22°C for 16 hours at 120 rpm. To the resultant culture liquid, there was added 2.5 ml of conversion mixture (2.5 mg/ml compactin sodium salt, 1 mg/ml FeSO₄•7H₂O, 10 mM NADPH, 50 % glycerol), and, thus, cultivation was continued at 22°C for 96 hours. The period of time which has passed after the addition of this conversion mixture is hereinafter referred to as "cultivation time". Then, to 100 µl of this culture liquid, there was added 100 µl of acetonitrile, and the resultant mixture was subjected to vortex for one minute at room temperature, and was then centrifuged for 10 minutes at 16,000 rpm with an Epfendorf centrifugator. So obtained supernatant was analyzed with HPLC, and, thus, there were detected pravastatin and other hydroxylated analogues which had been formed by the hydroxylation of substrate compactin.

Table 1 shows results of the measurement of the amount of pravastatin and RT-5.8 substance produced by static cells as mentioned in Example 3(1) with use of *Escherichia coli* transformant strain, under the protein induction condition of 18-28°C and with a conversion time of 48 hours.

**Table 1**

| Induction temperature | 18°C | | 22°C | | 25°C | | 28°C | |
|---|---|---|---|---|---|---|---|---|
| Hydroxylated compactin (µg/ml) | Pravastatin | RT5.8 substances | Pravastatin | RT5.8 substances | Pravastatin | RT5.8 substances | Pravastatin | RT5.8 substances |
| BL21(DE3)/ pET11a | 0 | 0 | 0 | 0 | 0 | 0 | 0 | 0 |
| BL21(DE3)/ pMoxAB | 0 | 0.18 | 0 | 0 | 0 | 0 | 0 | 0.3 |
| BL21(DE3)/ pMoxAB-fdr1 | 0.24 | 1.24 | 1.72 | 8.48 | 0.43 | 1.92 | 0 | 0.61 |
| BL21(DE3)/ pMoxAB-fdr2 | 0.25 | 1.31 | 2.15 | 10.21 | 0 | 0.84 | 0.35 | 1.65 |
| BL21(DE3)/ pMoxAB-camAB | 3.84 | 22.07 | 6.09 | 33.55 | 4.05 | 16.53 | 0.97 | 5.49 |

Productivity was the highest when induction temperature was 22°C, under which condition each of strains wherein *Streptomyces coelicolor* A3(2)-originated ferredoxin reductase *(fdr⁻ 1 or fdr⁻ 2)* had been co-expressed accumulated, in medium, 1.7 to 2.1 µg/ml of pravastatin and 8.4 to 10.2 µg/ml ofRT5.8 substances. A strain wherein *camAB* had been expressed showed much higher productivity; it accumulated, in medium, 6.09 µg/ml of pravastatin and 33.55 µg/ml ofRT5.8 substance. In the case where there was used, as control, vector alone (BL21(DE3)/pET11a) or a strain which contained no gene to encode ferredoxin reductase (BL21(DE3)/pMoxAB), there were hardly detected pravastatin and RT5.8 substances.

Table 2 shows results of test of productivity of pravastatin in Fed-batch method as mentioned in Example 4 (2).

**Table 2**

| Cultivation time | 4 hours | | 24 hours | | 48 hours | | 96 hours | |
|---|---|---|---|---|---|---|---|---|
| Hydroxylated compactin (µg/ml) | Pravastatin | RT5.8 substances | Pravastatin | RT5.8 substances | Pravastatin | RT5.8 substances | Pravastatin | RT5.8 substances |
| BL21(DE3)/ pET11a | 0 | 0 | 0 | 0 | 0 | 0 | 0 | 0 |
| BL21(DE3)/ pMoxAB | 0 | 0 | 0 | 0 | 0 | 0 | 0 | 0 |
| BL21Q)E3)/ pMoxAB-fdr1 | 0 | 0 | 0 | 0 | 0 | 0.87 | 0 | 0.61 |
| BL21(DE3)/ pMoxAB-fdr2 | 0 | 0 | 0 | 0 | 0 | 0.29 | 0 | 0.28 |
| BL21(DE3)/ pMoxAB-camAB | 0 | 0 | 0.28 | 2.82 | 0.64 | 7.74 | 0.95 | 12.44 |

In the results with cultivation time of 96 hours, strains wherein *Streptomyces coelicolor* A3(2)-originated ferredoxin reductase *(fdr⁻1* or *fdr⁻2)* had been co-expressed accumulated, in medium, 0.28 to 0.61 µg/ml of RD5.8 substances while accumulating no pravastatin. A strain wherein *camAB* had been expressed showed high productivity; it accumulated, in medium, 0.95 µg/ml of pravastatin and 12.44 µg/ml of RT5.8 substances. In the case where there was used, as control, vector alone (BL21(DE3)/pET11a) or a strain which contained no gene to encode ferredoxin reductase (BL21(DE3)/pMoxAB), pravastatin and RT5.8 substances were not detected.

In a strain wherein *camAB*had been co-expressed, *i.e*., in *Escherichia coli* wherein pMoxAB-camAB had been introduced, *moxB* (ferredoxin gene) and *camB* (putidaredoxin gene) among thus introduced genes overlap with each other in their function. In order to know which gene among the constituent genes contained in said pMoxAB-camAB are indispensable for the expression of activity, the inventors of this invention constructed a plasmid which lacked one or two of said constituent genes, and introduced the plasmid into *Escherichia coli.* Table 3 shows results of productivity of hydroxylated compactin with use of static cells of thus prepared strain, and with a conversion time of 24 hours.

**Table 3**

| | Constituent gene | | | | Hydroxylated compactin | |
|---|---|---|---|---|---|---|
| | (+ existent - non-exstent) | | | | (µg/ml) | |
| | moxA | moxB | camA | camB | Pravastatin | RT5.8 substances |
| BL21(DE3)/ pET11a | - | - | - | - | 0 | 0 |
| BL21(DE3)/ pMoxAB | + | + | - | - | 0 | 0 |
| BL21(DE3)/ pMoxAB-camA | + | + | + | - | 0.17 | 0.82 |
| BL21(DE3)/ pMoxA-camAB | + | - | + | + | 0 | 0 |
| BL21(DE3)/ pMoxAB-camAB | + | + | + | + | 1.67 | 9.96 |

The above shows that three kinds of gene of *moxA, moxB* and *camA* are essential for the expression of activity, and that the addition *of camB* achieves a remarkable increase in activity; the yield of hydroxylated compactin increased about 10 times.

### Example 5 Construction of plasmid

### (1) pT7NS-camAB

PCR was carried out under the following condition with use of primer PRR-1F
(5'-GCCCCCCATATGAACGCAAACGACAACGTGGTCATC-3') (see Sequence No. 9) and PRR-2R
(5'-GCGGATCCTCAGGCACTACTCAGTTCAGCTTTGGC-3') (see Sequence No. 10) by using, as a template, genomic DNA of *Pseudomonas putida* ATCC17453.

| (Composition of reaction liquid) | |
|---|---|
| Sterilized purified water | 15 µl |
| Twice-concentrated GC buffer I (Takara Shuzo) | 25 µl |
| dNTP mixed solution (dATP, dGTP, dTTP, dCTP each 2.5 mM) | 8 µl |
| PRR-1F primer (100 pmo1/µl) | 0.5 µl |
| PRR-2R primer (100 pmol/µl) | 0.5 µl |
| *Pseudomonas putida* ATCC 17453 | |
| Genomic DNA (10 ng/µl) | 0.5 µl |
| LA Taq (5 units/µl Takara Shuzo) | 0.5 µl |
| | |

| (Temperature condition) | |
|---|---|
| 95°C 3 minutes | |
| (98°C 20 seconds; 63°C 30 seconds; 68°C 2 minutes) 30 cycles | |
| 72°C 5 minutes | |

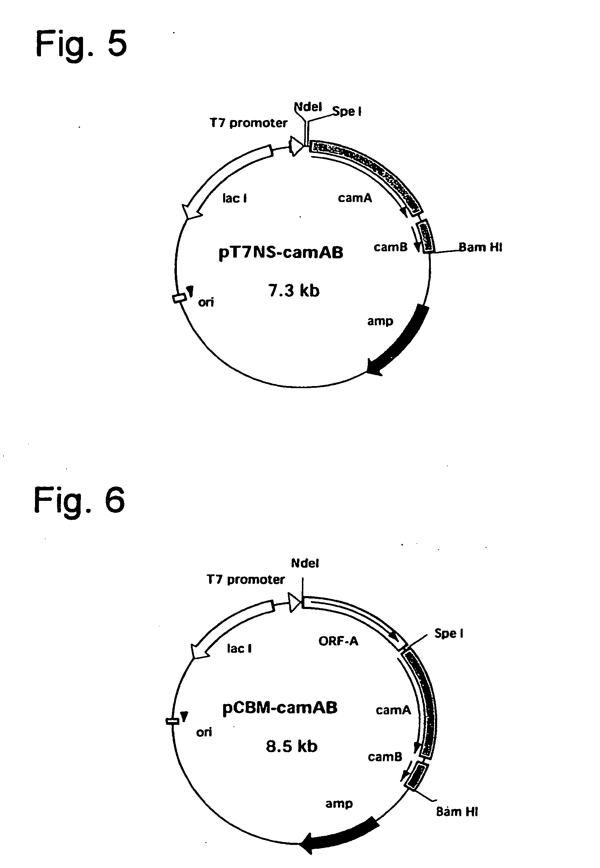

An amplified 1.5-kb fragment (*camAB*fragment) which contained ferredoxin reductase gene *(cam4)* and putidaredoxin gene (*camB*) just downstream thereto was treated with restriction enzyme *Nde* I and *Bam* HI, and was then subjected to electrophoresis in 0.8 % agarose gel. After the electrophoresis was over, *camAB*fragment was recovered, with use of SUPREC-01 (Takara Shuzo), from a gel piece containing said gene fragment which had been cut out from the gel, and was purified. Said fragment was ligated to *Nde*I site and *Bam* HI site of *Escherichia coli* plasmid vector pET11a (manufactured by Stratagene Co.) with use of T4 DNA ligase, and, then, *Escherichia coli* DH5α was transformed, and, thus, pT7-camAB was constructed. To *Nde* I site of said plasmid pT7-camAB, there was ligated by T4 DNA ligase one molecule of linker which had been prepared by the annealing of two kinds of synthetic oligo DNAs SP-1 (5'-TATGCGTCACTAGTCGGGAGTGCGTTA-3') (see Sequence No. 17) and SP-2 (5'-TATAACGCACTCCCGACTAGTGACGCA-3') (see Sequence No. 18), with which *Escherichia coli* DH5α was transformed, and, thus, plasmid pT7NS-camAB was constructed. Fig. 5 shows the structure of pT7NS-camAB.

### (2) pCBM-camAB

PCR was carried out under the following condition with use of primer CB-4F (5'-GCCCCCCATATGACAGCTTTGAATCTGATG-3') (see Sequence No. 19) and CB-5R
(5'-GCACTAGTCAGAGACGGACCGGCAGAC-3') (see Sequence No. 20) by using, as a template, total DNA of *Streptomyces thermotolerans* ATCC11416, and, thus, there was prepared 1.25 kb fragment of ORF-A (cytochrome P-450 gene which encodes enzyme to epoxidize 12- and 13-positions of carbomycin B) (gene sequence of ORF-A and the function of ORF-A are mentioned in Bioscience Biotechnology Biochemistry vol. 59, 582-588,1995).

| (Composition of reaction liquid) | |
|---|---|
| Sterilized purified water | 61 µl |
| 10 times-concentrated buffer (Takara Shuzo) | 10 µl |
| 25 mMgC12 | 10 µl |
| dNTP mixed solution (dATP, dGTP, dTTP, dCTP each 2.5 mM) | 16 µl |
| CB-4F primer (100 pmol/µl) | 0.5 µl |
| CB-5R primer (100 pmol/µl) | 0.5 µl |
| Total DNA (100 ng/µl) of *Streptomyces* | |
| *thermotolerans*ATCC11416 | 1µl |
| LA Taq (5 units/µl, Takara Shuzo) | 1 µl |
| | |

| (Temperature condition) | |
|---|---|
| 95°C 3 minutes | |
| (98°C 20 seconds; 63°C 30 seconds; 68°C 2 minutes) 30 cycles | |
| 72°C 5 minutes | |

This gene fragment was digested with restriction enzyme *Nde*I and *Spe*I, and was then subjected to electrophoresis in 0.8 % agarose gel. After the electrophoresis was over, ORF-A fragment was recovered, with use of *SUPREC-01 (Takara Shuzo), from a gel piece containing said gene fragment, which had been cut out from the gel, and was purified. Said fragment was ligated to *Nde* I*-Spe* I site of pT7NS-camAB with use of T4 DNA ligase, and, with the resultant reaction liquid, *Escherichia coli*DH5α was transformed, and, thus, plasmid pCBM-camAB was constructed. Fig. 6 shows the structure of pCBM-camAB.

### (3) pSC154A1-camAB

Total DNA of *Streptomyces coelicolor*A3(2) (imparted by John Innes Institute, Norwich, UK) was digested with restriction enzyme *Bam* HI and *PstI* to give a 100 ng/µl solution of TE (10 mM Tris-HCl [pH 8.0], 1 mM EDTA). PCR was carried out under the following condition by using this DNA as a template with use of primer 154A1-1F
(5'-GCCCCCCATATGGCGACCCAGCAGCCCGCCCTC-3') (see Sequence No. 21) and 154A1-2R
(5'-GCACTAGTCAGCCGGCGTGCA.GCAGGACCGG-3') (see Sequence No. 22), and, thus, there was prepared 1.2 kb gene fragment which encoded CYP154A1 (DNA sequence of gene which encodes *Streptomyces coelicolor* A3(2)-originated CYP154A1 has been published in gene database, e.g., Gen Bank, under gene name of SCE6.21).

| (Composition of reaction liquid) | |
|---|---|
| Sterilized purified water | 61 µl |
| 10 times-concentrated buffer (Takara Shuzo) | 10 µl |
| 25 mMgCl2 | 10 µl |
| dNTP mixed solution (dATP, dGTP, dTTP, dCTP each 2.5 mM) | 16 µl |
| 154A1-1F primer (100 pmol/pD | 0.5 µl |
| 154A1-2R primer (100 pmol/µl) | 0.5 µl |
| Total DNA (100 ng/µl) of *Streptomyces* | |
| *coelicolor*A3(2) digested with | |
| *Bam* HI*-Pst* I | 1 µl |
| LA Taq (5 units/µl, Takara Shuzo) | 1 µl |
| | |

| (Temperature condition) | |
|---|---|
| 95°C 3 minutes | |
| (98°C 20 seconds; 63°C 30 seconds; 68°C 2 minutes) 30 cycles | |
| 72°C 5 minutes | |

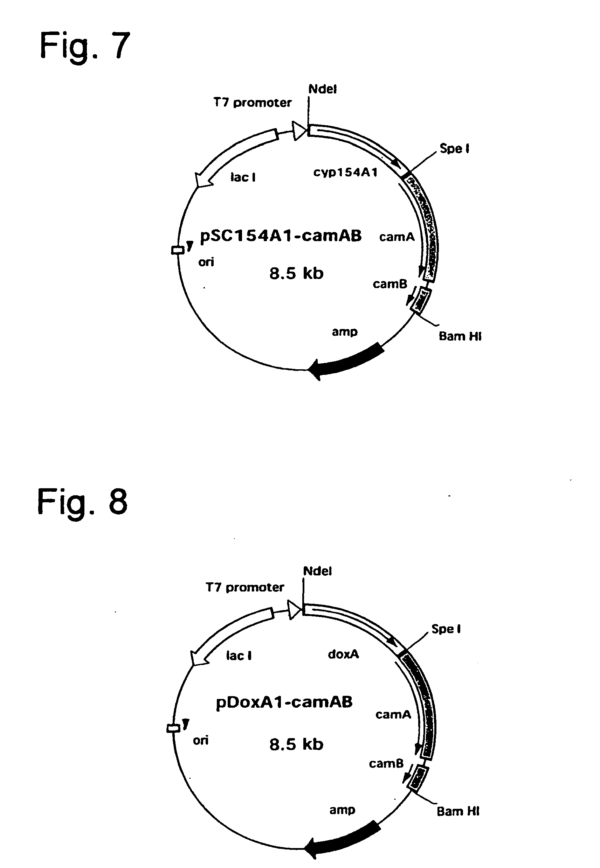

This gene fragment was digested with restriction enzyme *Nde*I and *Spe*I*,* and was then subjected to electrophoresis in 0.8 % agarose gel. After the electrophoresis was over, CYP154A1-encoding gene fragment was recovered, with use of SUPREC-01 (Takara Shuzo), from a gel piece containing said gene fragment which had been cut out from the gel, and was purified. Said fragment was ligated to *Nde*I*-Spe*I site of pT7NS-camAB with use of T4 DNA ligase, and, with the resultant reaction liquid, *Escherichia coli DH5α* was transformed, and, thus, plasmid pSC154A1-camAB was constructed. Fig. 7 shows the structure of pSC154A1-camAB.

### (4) pDoxA1-camAB

PCR was carried out under the following condition with use of primer DoxA-1F (5'-GCCCCCCATATGGCCGTCGACCCGTTCGCGTG-3') (see Sequence No. 23) and DoxA-2R
(5'-GCACTAGTCAGCGCAGCCAGACGGGCAGTTC-3') (see Sequence No. 24) by using, as a template, total DNA of daunomycin-producing bacterium *Streptomyces peucebus*ATCC 29050, and, thus, there was prepared 1.2-kb fragment of *doxA* (cytochrome P-450 gene which participates in the biosynthesis of daunomycin). DNA sequence of the *doxA* gene is mentioned in Journal of Bacteriology, vol. 181, No. 1, 305-318, 1999.

| (Composition of reaction mixture) | |
|---|---|
| Sterilized purified water | 61 µl |
| 10 times-concentrated buffer (Takara Shuzo) | 10 µl |
| 25 mMgC12 | 10 µl |
| dNTP mixed solution (dATP, dGTP, dTTP, dCTP each 2.5 mM) | 16 µl |
| DoxA-1F primer (100 pmol/µl) | 0.5 µl |
| DoxA-2R primer (100 pmol/µl) | 0.5 µl |
| Total DNA (100 ng/µl of *Streptomyces peuceticus* ATCC 29050 | 1 µl |
| LA Taq (5 units/µl, Takara Shuzo) | 1 µl |
| | |

| (Temperature condition) | |
|---|---|
| 95°C | 3 minutes |
| (98°C | 20 seconds; 63°C 30 seconds; 68°C 2 minutes) 30 cycles |
| 72°C | 5 minutes |

This gene fragment was digested with restriction enzyme *Nde*I and *Spe*I, and was then subjected to electrophoresis in 0.8 % agarose gel. After the electrophoresis was over, *doxA* fragment was recovered, with use of SUPREC-01 (Takara Shuzo), from a gel piece containing said gene fragment which had been cut out from the gel, and was purified. Said fragment was ligated to *Nde*I*-Spe*I site ofpT7NS-camAB with use of T4 DNAligase, and, with the resultant reaction liquid, *Escherichia coli* DH5α was transformed, and, thus, plasmid pDoxA1-camAB was constructed. Fig. 8 shows the structure of pDoxA1-camAB.

### Example 6 Microbial conversion with use of Escherichria coli recombinant wherein cytochrome P-450 had been expressed

### (1) Production of carbomycin A (for reference purposes)

Glycerol culture, in an amount of 10 µl, of *Escherichia coli* BL21(DE3) strain which had been transformed with pCBM-camAB among the plasmids as obtained in the above-mentioned Example 4 was added to 2 ml of LB medium to which 50 ug/ml (final concentration) of ampicillin had been added, and the resultant mixture was subjected to shake culture at 28°C for 16 hours at 220 rpm. Thus obtained culture liquid in an amount of 250 µl was added to 25 ml of NZCYM medium to which 50 ug/ml of ampicillin had been added, and the resultant mixture was subjected to shake culture at 37°C for 2.5 hours. Then, 25 µl of 100 mM IPTG and 25 µl of 80 mg/ml δ-amnolevulinic acid were added in order, and the resultant mixture was subjected to shake culture at 22°C at 120 rpm for 16 hours. Cells were recovered by centrifugation from the resultant culture liquid, and were then washed once with conversion buffer-2 (50 mM NaH₂PO₄, 1 mM EDTA, 0.2 mM DTT,10 % glycerol, [pH 7.31). Subsequently, the cells were suspended in 3 ml of said buffer to give a suspension of static cells. To 600 µl of this suspension of static cells, 3 µl of 100 mg/ml methanol solution of carbomycin B was added, and the resultant mixture was incubated at 28°C for five hours with shaking (220 rpm). Later, to thus obtained reaction liquid, there was added 100 µl of 50% K₂HPO₄ (pH 8.5) and 100 µl of ethyl acetate, and the resultant mixture was subjected to vortex, and then to centrifugation for five minutes at 16,000 rpm with an Epfendorf centrifugator. A TLC plate was spotted with 10 µl of so obtained ethyl acetate phase, and was then subjected to development with a developer (ethyl acetate : diethylamine = 100:2). Subsequently, this plate was sprayed with 10 % sulfuric acid, and heated at 100°C for 10 minutes. Spots on which color had come out were analyzed for coloring intensity with a dual-wavelength chromatoscanner CS-930 (manufactured by Shimadzu Seisakusho) at a wavelength of 600 nm, and, thus, there was evaluated the amount of carbomycin A (RF value in TLC: 0.64) which had been formed by the epoxidation of substrate carbomycin B (RF value in TLC: 0.71). As a result, it was confirmed that carbomycin A had been formed with a yield of 90 µg/ml. Then, substrate conversion reaction was conducted with use of a control strain BL21(DE3) (pET11a) under the same condition as stated above. As a result of analysis, no formation of carbomycin A was detected.

### (2) De-ethylation of 7-ethoxycoumarin

Glycerol culture, in an amount of 10 µl, of *Escherichia coli* BL21(DE3) strain which had been transformed with pSC154A1-camAB among the plasmids as obtained in the above-mentioned Example 5 was added to 2 ml of LB medium to which 50 µg/ml (final concentration) of ampicillin had been added, and the resultant mixture was subjected to shake culture at 28°C for 16 hours at 220 rpm. Thus obtained culture liquid in an amount of 250 µl was added to 25 ml of NZCYM medium to which 50 µg/ml of ampicillin had been added, and the resultant mixture was subjected to shake culture at 37°C for 2.5 hours. Then, 25 µl of 100 mM IPTG and 25 µl of 80 mg/ml δ-aminolevulinic acid were added in order, and the resultant mixture was subjected to shake culture at 22°C at 120 rpm for 16 hours. Cells were recovered by centrifugation from the resultant culture liquid, and were then washed once with conversion buffer2 (50 mM NaH₂PO₄, 1 mM EDTA, 0.2 mM DTT, 10 % glycerol, [pH 7.3]). Subsequently, the cells were suspended in 6 ml of said buffer to give a suspension of static cells. The 1 ml of this suspension of static cells, 5 µl of 50 mM DMSO solution of 7-ethoxycoumarin was added, and the resultant mixture was incubated at 28°C for five hours with shaking (220 rpm). Later, to thus obtained reaction liquid, there was added 200 µl of ethyl acetate, and the resultant mixture was subjected to vortex, and then to centrifugation for five minutes at 16,000 rpm with an Epfendorf centrifugator. There was taken out 100 µl of so obtained ethyl acetate phase, which was then evaporated to dryness in vacuum. The resultant dried pellet was dissolved in 1 ml of 100 mM potassium phosphate buffer (pH 7.4). Thus obtained solution was 80-times diluted, and was then measured for fluorescence (wavelength: 460 nm) with F-2000 spectrophotofluorometer (manufactured by Hitachi Science Systems, Co.) at an excitation wave length of 380 nm for the purpose of evaluation of the amount of 7-hydroxycoumarm which had been formed by de-ethylation of substrate 7-ethoxycoumarin. As a result, fluorescence intensity was 2770, and, thus, the formation of 7-hydroxycoumarin was confirmed. Then, substrate conversion reaction was conducted with use of a control strain BL21(DE3) (pET11a) under the same condition as stated above. As a result of analysis, fluorescence intensity was three or less.

### (3) Dehydrogenation of 13-dihydrodaunomycin

Glycerol culture, in an amount of 10 µl, of *Escherichia coli* BL21(DE3) strain which had been transformed by pDoxA1-camAB among the plasmids as obtained in the above-mentioned Example 5 was added to 2 ml of LB medium to which 50 ug/ml (final concentration) of ampicillin had been added, and the resultant mixture was subjected to shake culture at 28°C for 16 hours at 220 rpm. Thus obtained culture liquid in an amount of 250 µl was added to 25 ml of NZCYM medium to which 50 µg/ml of ampicillin had been added, and the resultant mixture was subjected to shake culture at 37°C for 2.5 hours. Then, 25 µl of 100 mM IPTG and 25 µl of 80 mg/ml δ-aminolevulinic acid were added in order, and the resultant mixture was subjected to shake culture at 22°C at 120 rpm for 24 hours. Cells were recovered by centrifugation from the resultant culture liquid, and were then washed once with conversion buffer-2 (50 mM NaH₂PO₄, 1 mM EDTA, 0.2 mM DTT, 10 % glycerol, [pH 7.3]). Subsequently, the cells were suspended in 4 ml of said buffer to give a suspension of static cells. To 1 ml of this suspension of static cells, 10 µl of 10 mg/ml methanol solution of 13-dihydrodaunomycin was added, and the resultant mixture was incubated at 28°C for 24 hours with shaking (220 rpm). Later, to 400 µl of thus obtained reaction liquid, there was added 1.2 ml of acetone, and the resultant mixture was subjected to vortex, and was then extracted with 300 µl of chloroform. Thus obtained extract was evaporated to dryness in vacuum, and was then dissolved in 500 µl of 0.3 M hydrochloric acid, and thus obtained solution was heated at 80°C for 30 minutes. This solution was extracted with 100 µl of chloroform, and thus obtained extract was evaporated to dryness in vacuum. The resultant dried pellet was dissolved in 100 µl of methanol, and the resultant solution was subjected to HPLC under the following condition, and, thus, there was detected daunomycin which had been formed by the dehydrogenation of substrate 13-dihydrodaunomycin.

### (Analytical condition of HPLC)

| | | |
|---|---|---|
| Analytical apparatus: | Shimadzu LC10 Chromatopac (manufactured by Shimadzu Seisakusho) | |
| Column: ZORBAX TMS (5 µ) 4.6 mm × 250 mm I.D. | | |
| Mobile phase: | | |
| To a mixture of water/acetonitrile/methanol/phosphoric acid = | | |
| 540:290:170:2 (volume ratio), 1.0 g of sodium lauryl sulfate was | | |
| added and dissolved, and, to the resultant mixture, 2N NaOH was | | |
| added for the adjustment of pH to 3.6. | | |
| Flow rate: | 1.5 ml/minute | |
| Wavelength for detection: | 254 nm | |
| Injection content: | 20 µl | |
| Column temperature: | 40°C | |
| Analysis time: | 20 minutes | |
| Retention time: | 13-dihydrodaunomycin | 4.8 minutes |
| | daunomycin | 5.9 minutes |

Analysis detected 3.7 µg/ml of daunomycin. Then, substrate conversion reaction was conducted with use of a control strain BL21(DE3) (pET11a) under the same condition as stated above. As a result of analysis, no formation of daunonycin was detected.

### Industrial applicability

In accordance with this invention, single oxygen atom insertional reaction of organic compound as a substrate can efficiently be conducted with use of a recombinant which has been constructed by use of actinomycete cytochrome P-450 gene and *Escherichia coli as* a host.

## Claims

1. A system for the expression of actinomycete cytochrome P-450 genes in host *Escherichia coli,* wherein said *Escherichia. coli* contains a recombinant DNA molecule which comprises xenogenic microorganism-originated ferredoxin gene, ferredoxin reductase gene as well as said cytochrome P-450 gene, in operable state, wherein ferredoxin gene and ferredoxin reductase gene are respectively putidaredoxin gene (*camB*) and putidaredoxin reductase gene (*camA*) which are each originated from *Pseudomonas putida.*

2. A system for the expression of actinomycete cytochrome P-450 genes in host *Escherichia coli,* wherein said *Escherichia coli* contains a recombinant DNA molecule which comprises xenogenic microorganism-originated ferredoxin gene, ferredoxin reductase gene as well as said cytochrome P-450 gene, in operable state, wherein actinomycete cytochrome P-450 gene and ferredoxin gene are originated from one and the same gene cluster of actinomycete, in which ferredoxin reductase gene is putidaredoxin reductase gene (*camA*) originated from *Pseudomonas putida,* and which further contains, as another ferredoxin gene, putidaredoxin gene (*camB*) originated from *Pseudomonas putida.*

3. An expression system of claim 1 or 2 wherein actinomycete cytochrome P-450 gene is a compactin-hydroxylating enzyme-encoding gene (*moxA*) originated from *Microtetraspora recticatena.*

4. A system for the expression of actinomycete cytochrome P-450 genes in host *Escherichia coli,* wherein said *Escherichia coli* contains a recombinant DNA molecule which comprises xenogenic microorganism-originated ferredoxin gene, ferredoxin reductase gene as well as said cytochrome P-450 gene, in operable state, wherein actinomycete cytochrome P-450 gene and ferredoxin gene are respectively compactin hydroxylating enzyme encoding gene (*moxA*) originated from *Microtetraspora recticatena* and ferredoxin gene (*moxB*) which is adjacent downstream to *moxA.*

5. An expression system of claim 4 wherein ferredoxin reductase gene is ferredoxin reductase gene *fdr-1* or *fdr-2* originated from *Streptomyces coelicolor.*

6. An expression system of claim 4 wherein said expression system further contains, as another ferredoxin gene, putidaredoxin gene (*camB*) originated from *Pseudomonas putida,* and in which ferredoxin reductase gene is putidaredoxin reductase gene *(camA)* originated from *Pseudomonas putida.*

7. An expression system according to any one of claims 1 to 6 in which the induction of expression of cytochrome P-450 gene is conveniently carried out at 20 to 24°C.

8. An expression system according to any one of claims 1 to 7 wherein said cytochrome P-450 gene comprises polynucleotide which is selected from the group consisting of polynucleotide having a continuous nucleotide sequence from base 313 to base 1533 in Sequence No. 1 or functionally equivalent polynucleotide with homology of at least 80 % to said nucleotide sequence, and polynucleotide having a continuous nucleotide sequence from base 544 to base 1758 in Sequence No. 2 or functionally equivalent polynucleotide with homology of at least 80% to said nucleotide sequence, and wherein a functionally equivalent polynucleotide has activity to catalyze single oxygen atom insertional reaction against corresponding substrates in the expression system.

9. An expression system according to any one of claims 1 to 8 wherein said ferredoxin gene comprises polynucleotide having a continuous nucleotide sequence from base 1439 to base 1759 in Sequence No. 16 or functionally equivalent polynucleotide with homology of at least 80% to said nucleotide sequence and which has activity to catalyze single oxygen atom insertional reaction against corresponding substrates in the expression system.

10. An expression system according to any one of claims 1 to 9 wherein said ferredoxin reductase gene comprises polynucleotide having a continuous nucleotide sequence from base 115 to base 1380 in Sequence No. 16 or functionally equivalent polynucleotide with homology of at least 80% to said nucleotide sequence and which has activity to catalyze single oxygen atom insertional reaction against corresponding substrates in the expression system.

11. Use of the expression system according to any one of claims 4 to 6 for introducing a hydroxyl group at 6β-position of compactin.

## Patentansprüche

1. System für die Expression von Actinomyceten-Cytochrom P-450-Genen in *Escherichia coli* als Wirt, wobei die *Escherichia coli* ein rekombinantes DNA-Molekül enthält, welches von einem xenogenen Mikroorganismus stammendes Ferredoxin-Gen, Ferredoxin-Reduktase-Gen sowie das Cytochrom-P450-Gen in einem funktionellen Zustand umfasst, wobei Ferredoxin-Gen und Ferredoxin-Reduktase-Gen Putidaredoxin-Gen (*camB*) bzw. Putidaredoxin-Reduktase-Gen (*camA*) sind, welche von *Pseudomonas putida* stammen.

2. System für die Expression von Actinomyceten-Cytochrom P-450-Genen in *Escherichia coli* als Wirt, wobei die *Escherichia coli* ein rekombinantes DNA-Molekül enthält, welches von einem xenogenen Mikroorganismus stammendes Ferredoxin-Gen, Ferredoxin-Reduktase-Gen sowie das Cytochrom-P450-Gen in einem funktionellen Zustand umfasst, wobei das Actinomyceten-Cytochrom P-450-Gen und das Ferredoxin-Gen von ein und demselben Actinomyceten-Gen-Cluster stammen, in welchem Ferredoxin-Reduktase-Gen Putidaredoxin-Reduktase-Gen (*camA*), das von *Pseudomonas putida* stammt, ist und welches außerdem als weiteres Ferredoxin-Gen Putidaredoxin-Gen (*camB*), das von *Pseudomonas putida* stammt, enthält.

3. Expressionssystem gemäß Anspruch 1 oder 2, wobei das Actinomyceten-Cytochrom P450-Gen ein für Compactinhydroxylierendes Enzym-codierendes Gen (*moxA*), das von *Microtetraspora recticatena* stammt, ist.

4. System für die Expression von Actinomyceten-Cytochrom P-450-Genen in *Escherichia coli* als Wirt, wobei die *Escherichia coli* ein rekombinantes DNA-Molekül enthält, welches von einem xenogenen Mikroorganismus stammendes Ferredoxin-Gen, Ferredoxin-Reduktase-Gen sowie das Cytochrom-P450-Gen in einem funktionellen Zustand umfasst, wobei das Actinomyceten-Cytochrom P-450-Gen und das Ferredoxin-Gen für Compactinhydroxylierendes Enzym-codierendes Gen (*moxA*), das von *Microtetraspora recticatena* stammt, bzw. Ferredoxin-Gen (*moxB*), das stromabwärts benachbart zu *moxA* ist, sind.

5. Expressionssystem gemäß Anspruch 4, wobei das Ferredoxin-Reduktase-Gen Ferredoxin-Reduktase-Gen *fdr-1* oder *fdr-2,* das von *Streptomyces coelicolor* stammt, ist.

6. Expressionssystem gemäß Anspruch 4, wobei das Expressionssystem außerdem als weiteres Ferredoxin-Gen Putidaredoxin-Gen (*camB*), das von *Pseudomonas putida* stammt, enthält und wobei das Ferredoxin-Reduktase-Gen Putidaredoxin-Reduktase-Gen (*camA*), das von *Pseudomonas putida* stammt, ist.

7. Expressionssystem gemäß einem der Ansprüche 1 bis 6, wobei die Induktion der Expression von Cytochrom P-450-Gen zweckmäßigerweise bei 20 bis 24°C durchgeführt wird.

8. Expressionssystem gemäß einem der Ansprüche 1 bis 7, wobei das Cytochrom P450-Gen ein Polynucleotid umfasst, welches ausgewählt ist aus der Gruppe, bestehend aus einem Polynucleotid, das eine fortlaufende Nucleotidsequenz von Base 313 bis Base 1533 in Sequenz Nr. 1 hat, oder einem funktionell äquivalenten Polynucleotid mit wenigstens 80% Homologie zu der genannten Nucleotidsequenz und einem Polynucleotid, das eine fortlaufende Nucleotidsequenz von Base 544 bis Base 1758 in Sequenz Nr. 2 hat, oder einem funktionell äquivalenten Polynucleotid mit wenigstens 80% Homologie zu der genannten Nucleotidsequenz, und wobei ein funktionell äquivalentes Polynucleotid Aktivität hat, um eine Insertionsreaktion eines einzelnen Sauerstoffatoms in entsprechende Substrate in dem Expressionssystem zu katalysieren.

9. Expressionssystem gemäß einem der Ansprüche 1 bis 8, wobei das Ferredoxin-Gen ein Polynucleotid, das eine fortlaufende Nucleotidsequenz von Base 1439 bis Base 1759 in Sequenz Nr. 16 hat, oder ein funktionell äquivalentes Polynucleotid mit wenigstens 80% Homologie zu der genannten Nucleotidsequenz umfasst, welches Aktivität hat, um eine Insertionsreaktion eines einzelnen Sauerstoffatoms in entsprechende Substrate in dem Expressionssystem zu katalysieren.

10. Expressionssystem gemäß einem der Ansprüche 1 bis 9, wobei das Ferredoxin-Reduktase-Gen ein Polynucleotid, das eine fortlaufende Nucleotidsequenz von Base 115 bis Base 1380 in Sequenz Nr. 16 hat, oder ein funktionell äquivalentes Polynucleotid mit wenigstens 80% Homologie zu der genannten Nucleotidsequenz umfasst, welches Aktivität hat, um eine Insertionsreaktion eines einzelnen Sauerstoffatoms in entsprechende Substrate in dem Expressionssystem zu katalysieren.

11. Verwendung des Expressionssystems gemäß einem der Ansprüche 4 bis 6 zur Einführung einer Hydroxylgruppe in 6β-Position von Compactin.

## Revendications

1. Un système d'expression des gènes codants pour le cytochrome P-450 d'origine d'actinomycète dans *Escherichia coli* comme hôte, dans lequel *Escherichia coli* contient une molécule d'ADN recombinant qui comprend en état fonctionnel le gène de la ferrédoxine, le gène de la ferrédoxine réductase et le gène du cytochrome P-450 dérivés d'un microorganisme xénogénique, dans lequel le gène de la ferrédoxine et le gène de la ferrédoxine réductase sont le gène de la putidarédoxine (*camB*) et le gène de la putidarédoxine réductase (*camA*), chacun dérivé de *Pseudomonas putida.*

2. Un système d'expression des gènes codants pour le cytochrome P-450 d'origine d'actinomycète dans *Escherichia coli* comme hôte, dans lequel *Escherichia coli* contient une molécule d'ADN recombinant qui comprend en état fonctionnel le gène de la ferrédoxine, le gène de la ferrédoxine réductase et le gène du cytochrome P-450 dérivés d'un microorganisme xénogénique, dans lequel le gène du cytochrome P-450 d'origine actinomycète et le gène de la ferrédoxine sont dérivés d'un seul et même cluster de gènes d'origine actinomycète, dans lequel le gène de la ferrédoxine réductase est le gène de la putidarédoxine réductase (*camA*) dérivé de *Pseudomonas putida,* et qui comprend en outre le gène de la putidarédoxine (*camB*) dérivé de *Pseudomonas putida* en tant qu'autre gène de la ferrédoxine.

3. Le système d'expression selon la revendication 1 ou 2, dans lequel le gène du cytochrome P-450 est un gène (*moxA*) codant pour l'hydroxylase de la compactine et dérivé de *Microtetraspora recticatena.*

4. Un système d'expression des gènes codants pour le cytochrome P-450 d'origine actinomycète dans *Escherichia coli* comme hôte, dans lequel *Escherichia coli* contient une molécule d'ADN recombinant qui comprend en état fonctionnel le gène de la ferrédoxine, le gène de la ferrédoxine réductase et le gène du cytochrome P-450 dérivés d'un microorganisme xénogénique, dans lequel le gène du cytochrome P-450 d'origine actinomycète et le gène de la ferrédoxine sont le gène (*moxA*) codant pour l'hydroxylase de la compactine et dérivé de *Microtetraspora recticatena* et le gène (*moxB*) de la ferrédoxine qui est voisin en aval de *moxA.*

5. Le système d'expression selon la revendication 4, dans lequel le gène de la ferrédoxine réductase est le gène de la ferrédoxine réductase *fdr-1* ou *fdr-2* dérivé de *Streptomyces coelicolor.*

6. Le système d'expression selon la revendication 4 comprenant en outre le gène (*camB*) de la putidarédoxine dérivé de *Pseudomonas putida* en tant qu'autre gène de la ferrédoxine et dans lequel le gène de la ferrédoxine réductase est le gène *(camA)* de la putidarédoxine réductase dérivé de *Pseudomonas putida.*

7. Le système d'expression selon l'une quelconque des revendications 1 à 6, dans lequel l'induction d'expression du gène du cytochrome P-450 est effectuée de préférence de 20 à 24°C.

8. Le système d'expression selon l'une quelconque des revendications 1 à 7, dans lequel le gène du cytochrome P-450 comprend un polynucléotide qui est choisi parmi le groupe consistant en un polynucléotide ayant une séquence nucléotidique continue de la base 313 à la base 1533 dans la Séquence Numéro 1 ou un polynucléotide fonctionnellement équivalent présentant au moins 80% d'homologie avec ladite séquence nucléotidique et un polynucléotide ayant une séquence nucléotidique continue de la base 544 à la base 1758 dans la Séquence Numéro 2 ou un polynucléotide functionnellement équivalent présentant au moins 80% d'homologie avec ladite séquence nucléotidique et dans lequel un polynucléotide fonctionnellement équivalent possède l'activité pour catalyser une réaction d'insertion d'un seul atome d'oxygène à des substrats appropriés dans le système d'expression.

9. Le système d'expression selon l'une quelconque des revendications 1 à 8, dans lequel le gène de la ferrédoxine comprend un polynucléotide ayant une séquence nucléotidique continue de la base 1439 à la base 1759 dans la Séquence Numéro 16 ou un polynucléotide fonctionnellement équivalent présentant au moins 80% d'homologie avec ladite séquence nucléotidique qui possède l'activité pour catalyser une réaction d'insertion d'un seul atome d'oxygène à des substrats appropriés dans le système d'expression.

10. Le système d'expression selon l'une quelconque des revendications 1 à 9, dans lequel le gène de la ferrédoxine réductase comprend un polynucléotide ayant une séquence nucléotidique continue de la base 115 à la base 1380 dans la Séquence Numéro 16 ou un polynucléotide fonctionnellement équivalent présentant au moins 80% d'homologie avec ladite séquence nucléotidique qui possède l'activité pour catalyser une réaction d'insertion d'un seul atome d'oxygène à des substrats appropriés dans le système d'expression.

11. Utilisation du système d'expression selon l'une quelconque des revendications 4 à 6 pour introduire un groupe hydroxyle à la 6ß-position de la compactine.
